# EUROPEAN PATENT APPLICATION

(11) **EP 4 510 147 A1**
(43) Date of publication of application: **19.02.2025**
(21) Application number: 24193056.9
(22) Date of filing: 06.08.2024
(51) Int. Cl.: G16H 40/63, G16H 50/20, G16H 50/70

(54) **PREDICTION OF A PHYSIOLOGICAL PARAMETER**

(30) Priority: 15.08.2023 US 202363519722 P; 22.07.2024 US 202418780414
(71) Applicant: MEDTRONIC MINIMED, INC., Northridge, CA 91325-1219 (US)
(72) Inventor: ABRAHAM, Sinu Bessy, Northridge, 91325 (US); KAFKA, Gene T., Northridge, 91325 (US); MIKHNO, Arthur, Northridge, 91325 (US); ZHONG, Yuxiang, Northridge, 91325 (US)
(74) Representative: Zimmermann & Partner Patentanwälte mbB

(57) **Abstract**

Disclosed herein are techniques related to predicting a physiological condition of a user. In some embodiments, the techniques may involve obtaining one or more glucose concentration values measured from a user; applying, to the one or more glucose concentration values measured from the user, a first glucose prediction model for a first prediction horizon; obtaining, based on applying the first glucose prediction model, a first predicted glucose value of the user; and predicting a second predicted glucose value of the user for a second prediction horizon that is less than the first prediction horizon, based on the first predicted glucose value and at least one glucose concentration value of the one or more glucose concentration values. In some scenarios, the physiological condition may include, for example, hypoglycemia or hyperglycemia.

## Description

### CROSS-REFERENCE TO RELATED APPLICATIONS

This application claims the benefit of priority to U.S. Provisional Application No. 63/519,722, filed August 15, 2023, entitled "PREDICTION OF PHYSIOLOGICAL PARAMETER" which is incorporated herein by reference in its entirety.

### TECHNICAL FIELD

The present disclosure relates generally to predicting a physiological parameter (e.g., blood glucose level) of a user, and more particularly to using machine learning estimations as a basis for prediction of the physiological parameter at a prediction horizon.

### BACKGROUND

Monitoring one's blood glucose level can be an important part of managing certain physiological conditions. For example, a diabetic patient or a user may use a diabetes management device to monitor blood glucose level in order to provide insulin therapy to prevent undesirable outcomes such as hypoglycemia or hyperglycemia. It is useful to be able to predict one's blood glucose level and conditions arising therefrom in the future to further facilitate management of such conditions.

### SUMMARY

Disclosed herein are techniques related to predicting a physiological parameter. The techniques may be practiced in a variety of ways, such as using a processor-implemented method; a system including one or more processors and one or more processor-readable media; and/or one or more (non-transitory) processor-readable media.

In some aspects of the present disclosure, a processor-implemented method is disclosed. In some embodiments, the techniques of the processor-implemented method may involve: obtaining one or more glucose concentration values measured from a user; applying, to the one or more glucose concentration values measured from the user, a first glucose prediction model for a first prediction horizon; obtaining, based on applying the first glucose prediction model, a first predicted glucose value of the user; and predicting a second predicted glucose value of the user for a second prediction horizon that is less than the first prediction horizon, based on the first predicted glucose value and at least one glucose concentration value of the one or more glucose concentration values.

In some aspects of the present disclosure, a system is disclosed. In some embodiments, the system may include one or more processors; and one or more processor-readable media storing instructions which, when executed by the one or more processors, cause performance of: obtaining one or more glucose concentration values measured from a user; estimating, using the one or more glucose concentration values, a first blood glucose value of the user within a predetermined length of time; obtaining a prediction horizon that is less than the predetermined length of time, a first threshold level, and a second threshold level; estimating a second blood glucose value of the user within the prediction horizon based on the first blood glucose value and a first machine learning model trained to determine whether a blood glucose level of the user will be lower than the first threshold level within the prediction horizon, or based on the first blood glucose value and a second machine learning model trained to determine whether the blood glucose level of the user will be higher than the second threshold level within the prediction horizon; and generating a prediction of a physiological condition of the user based at least on the estimated second blood glucose value.

In some aspects of the present disclosure, one or more non-transitory processor-readable media are disclosed. In some embodiments, the one or more non-transitory processor-readable media may store instructions which, when executed by one or more processors, cause performance of: obtaining one or more glucose concentration values measured from a user; applying, to the one or more glucose concentration values measured from the user, a first glucose prediction model for a first prediction horizon; obtaining, based on applying the first glucose prediction model, a first predicted glucose value of the user; and predicting a second predicted glucose value of the user for a second prediction horizon that is less than the first prediction horizon, based on the first predicted glucose value and at least one glucose concentration value of the one or more glucose concentration values.

Further disclosed herein are techniques related to predicting a physiological condition of a user. In some embodiments, the techniques may involve obtaining one or more glucose concentration values measured from a user; applying, to the one or more glucose concentration values measured from the user, a first glucose prediction model for a first prediction horizon; obtaining, based on applying the first glucose prediction model, a first predicted glucose value of the user; and predicting a second predicted glucose value of the user for a second prediction horizon that is less than the first prediction horizon, based on the first predicted glucose value and at least one glucose concentration value of the one or more glucose concentration values. In some scenarios, the physiological condition may include, for example, hypoglycemia or hyperglycemia.

In some scenarios, the physiological condition may include, for example, hypoglycemia or hyperglycemia.

### BRIEF DESCRIPTION OF THE DRAWINGS

The above and other aspects and features of the disclosure will become more apparent in view of the following detailed description when taken in conjunction with the accompanying drawings wherein like reference numerals identify like elements.
FIG. 1 is a diagram of an example therapy delivery system, in accordance with aspects of the present disclosure.
FIGS. 2A and 2B illustrate examples of machine learning (ML) models that are trained to predict a future physiological parameter after a period of time, according to some embodiments.
FIG. 3 is a diagram of an example of an architecture having a predictive system, according to some embodiments.
FIG. 4 is an example of a graph illustrating interpolation to determine a potential hypoglycemic indication.
FIGS. 5A - 5C illustrate examples of applications of a "hold" approach for producing a raw hyperglycemia indication or a raw hypoglycemia indication.
FIG. 6 is a flow diagram illustrating an example of a method for predicting a physiological condition of a user.
FIG. 7 is a flow diagram illustrating another example of a method for predicting a physiological condition of a user.
FIG. 8 is a flow diagram illustrating another example of a method for predicting a physiological condition of a user.
FIG. 9 is a diagram of an example of an insulin delivery device, in accordance with aspects of the present disclosure.
FIG. 10 is a block diagram of an example of a computer system, which can be utilized in embodiments described herein.

### DETAILED DESCRIPTION

It is important for a user (including a caretaker) to be aware of changes and anomalies in physiological parameters of a patient, such as the patient's blood glucose level. For example, for a diabetes patient in insulin therapy scenarios, delivering a therapeutic substance (e.g., an insulin bolus dosage) when or before, e.g., blood glucose spikes can help manage the glucose level in the body. As another example, consuming carbohydrates or a meal can restore a blood glucose level that has fallen and counteract associated symptoms.

For reasons such as these, a mechanism that can predict hypoglycemia and hyperglycemia at a certain future time or future times can advantageously, e.g., alert users and caretakers ahead of time. One or more machine learning models and/or numerical analytical techniques can be used to estimate a physiological parameter (e.g., blood glucose level) for a prediction horizon (e.g., some amount of time into the future). Users can then avoid such hypoglycemic and hyperglycemic episodes and maintain good glycemic control by suitably intervening before such episodes occur. Moreover, as a prediction mechanism, it is desirable that users can have flexibility in deciding thresholds or conditions that indicate hypoglycemia and hyperglycemia. Different users may wish to be alerted based on differing thresholds for hypoglycemia or hyperglycemia (or differing ranges for normal glucose levels).

Moreover, a predictive alert mechanism should be reliable in its predictions. The predictive alert mechanism should reduce false alerts (false positives) while maintaining appropriate sensitivity so as not to miss correct positive predictions while reducing false alerts. This can help to reduce alert fatigue (where users become desensitized to the alerts, leading to missed or ignored alerts or delayed responses), improve the patient experience, and gain user confidence to act upon the predictive alerts to prevent adverse conditions.

According to certain embodiments and techniques disclosed herein, machine learning models may be used with a predictive system to predict a patient's blood glucose levels in the future. More specifically, a threshold may be selected for determining whether the predicted blood glucose level of the patient is indicative of a physiological condition, such as hyperglycemic or hypoglycemic, over a future time period (prediction horizon such as 60 minutes). If the patient's blood glucose level is predicted to be below a certain threshold, an indication of hypoglycemia may be generated, and *vice versa* for hyperglycemia. In some embodiments, intermediate time periods can be selected and the corresponding glucose levels may be determined to provide a more granular result as to the patient's blood glucose level or predictions of the physiological condition. In one approach, an analytical technique can be applied to the predicted blood glucose level for a prediction horizon and a current sensor glucose measurement to estimate glucose levels for different prediction horizons. For example, interpolation techniques can be applied between said blood glucose level and sensor glucose measurement to generate a prediction for a second prediction horizon (e.g., 30 minutes), which can also be compared against thresholds to predict whether the patient will be in hyperglycemia or hypoglycemia within that second prediction horizon. In some embodiments, before generating a final alert for the predictions, these indications can be further evaluated and qualified to reduce false positives or otherwise provide a more accurate prediction. For example, in some implementations, some indications may be "held" until further indications (e.g., two or three consecutive triggering predictions) validate the prediction. In some implementations, contextual information or user settings can also inform the indication before generating a final indication, which may manifest as an alert.

Discussions utilizing terms such as, for example, "processing," "computing," "calculating," "determining," "establishing," "analyzing," "checking," or the like, may refer to operation(s) and/or process(es) of a computer, a computing platform, a computing system, or other electronic computing device, that manipulate and/or transform data represented as physical (e.g., electronic) quantities within the computer's registers and/or memories into other data similarly represented as physical quantities within the computer's registers and/or memories or other non-transitory information storage media that may store instructions to perform operations and/or processes by, e.g., one or more processor or processor apparatus (e.g., system on a chip) or a device associated with such processor(s).

Unless explicitly stated, the methods described herein are not constrained to a particular order or sequence. Additionally, some of the described methods or elements thereof can occur or be performed simultaneously or concurrently.

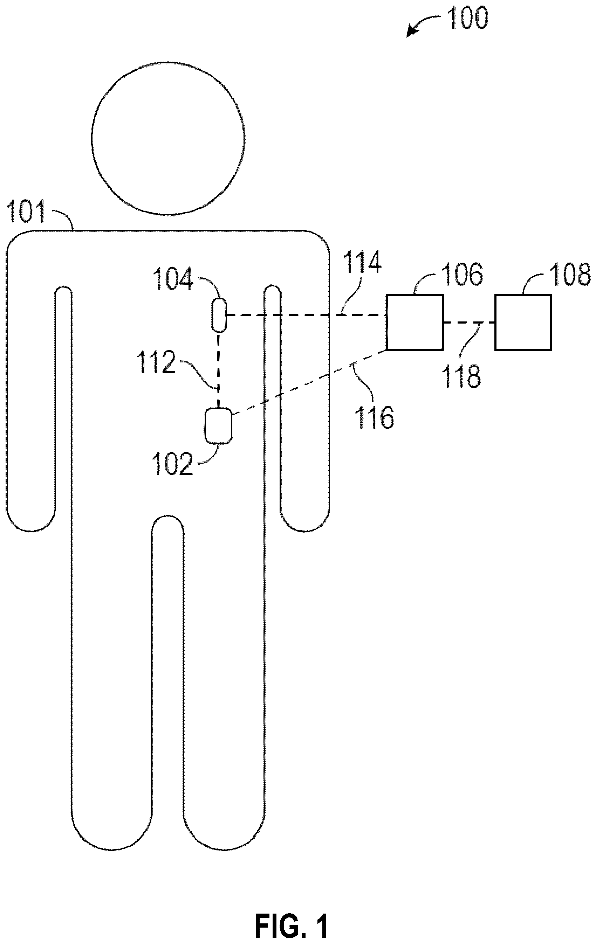

FIG. 1 depicts an example therapy delivery system 100 for a person 101. System 100 may be an insulin delivery system. The depicted therapy delivery system 100 includes a delivery device 102, a monitoring device 104, a computing device 106, and an optional remote or cloud computing system 108. The delivery device 102, the monitoring device 104, and the computing device 106 may be embodied in various ways, including being disposed in one or more device housings. For example, in some embodiments, all of the devices 102-106 may be disposed in a single device housing. In some embodiments, each of the devices 102-106 may be disposed in a separate device housing. In some embodiments, two or more of the devices 102-106 may be disposed in the same device housing, and/or a single device 102, 104, or 106 may have two or more parts that are disposed in two or more housings. Such embodiments, and combinations thereof, are contemplated to be within the scope of the present disclosure.

FIG. 1 also depicts communications links 112-118. The communications links 112-118 may each be a wired connection and/or a wireless connection. In the case where two devices are located in the same device housing, the communication link may include, for example, wires, cables, and/or communication buses on a printed circuit board, among other things. In the case where two devices are separated from each other in different device housings, the communication links may be wired and/or wireless connections. Wired connections may include, without limitation, an Ethernet connection, a USB connection, and/or another type of physical connection. Wireless connections may include, without limitation, a cellular connection, a Wi-Fi connection, a Bluetooth^{®} connection, a mesh network connection, and/or another type of connection using a wireless communication protocol. Some embodiments of the communication links 112-118 may use direct connections, such as Bluetooth^{®} connections, and/or may use connections that route through one or more networks or network devices (not shown), such as an Ethernet network, a Wi-Fi network, a cellular network, a satellite network, an intranet, an extranet, the Internet, and/or the Internet backbone, among other types of networks. Various combinations of wired and/or wireless connections may be used for the communication links 112-118.

Aspects of the insulin delivery system 100 are described below. Further aspects and details may be described in United States Patent Nos.: 4,562,751; 4,685,903; 5,080,653; 5,505,709; 5,097,122; 6,485,465; 6,554,798; 6,558,320; 6,558,351; 6,641,533; 6,659,980; 6,752,787; 6,817,990; 6,932,584; and 7,621,893. The entire contents of each of the foregoing United States Patents are hereby incorporated by reference herein.

The delivery device 102 is configured to deliver a therapeutic substance (e.g., insulin) to a person 101. The delivery device 102 may be secured to the person 101 (e.g., to the body or clothing of the person 101) or may be implanted on or in the body of the person 101. In some embodiments, the delivery device 102 may include a reservoir, an actuator, a delivery mechanism, and a cannula (not shown). The reservoir may be configured to store an amount of the therapeutic substance. In some embodiments, the reservoir may be refillable or replaceable. The actuator may be configured to drive the delivery mechanism. In some examples, the actuator may include a motor, such as an electric motor. The delivery mechanism may be configured to move the therapeutic substance from the reservoir through the cannula. In some examples, the delivery mechanism may include a pump and/or a plunger. The cannula may facilitate a fluidic connection between the reservoir and the body of the person 101. The cannula and/or a needle may facilitate delivery of the therapeutic substance to a tissue layer, vein, or body cavity of the person 101. During operation, the actuator, in response to a signal (e.g., a command signal), may drive the delivery mechanism, thereby causing the therapeutic substance to move from the reservoir, through the cannula, and into the body of the person 101.

The components of the delivery device 102 described above are merely provided as examples. The delivery device 102 may include other components, such as, without limitation, a power supply, a communication transceiver, computing resources, and/or user interfaces, among other things. Persons skilled in the art will recognize various implementations of the delivery device 102 and the components of such implementations. All such implementations and components are contemplated to be within the scope of the present disclosure.

With continuing reference to FIG. 1, the monitoring device 104 is configured to detect a physiological condition (e.g., a glucose concentration level) of the person 101 and may also be configured to detect other things. The monitoring device 104 may be secured to the body of the person 101 (e.g., to the skin of person 101 via an adhesive) and/or may be at least partially implanted into the body of the person 101. Depending on the particular location or configuration, the monitoring device 104 may be in contact with biological matter (e.g., interstitial fluid and/or blood) of the person 101.

The monitoring device 104 includes one or more sensors (not shown), such as, without limitation, electrochemical sensors, electrical sensors, and/or optical sensors. As persons skilled in the art will understand, an electrochemical sensor may be configured to respond to the interaction or binding of a biological marker to a substrate by generating an electrical signal based on a potential, conductance, and/or impedance of the substrate. The substrate may include a material selected to interact with a particular biomarker, such as glucose. The potential, conductance, and/or impedance may be proportional to a concentration of the particular biomarker. In the case of electrical sensors, and as persons skilled in the art will understand, an electrical sensor may be configured to respond to an electrical biosignal by generating an electrical signal based on an amplitude, frequency, and/or phase of the electrical biosignal. The electrical biosignal may include a change in electric current produced by the sum of an electrical potential difference across a tissue, such as the nervous system, of the person 101. In some embodiments, the electrical biosignal may include portions of a potential change produced by the heart of the person 101 over time, e.g., recorded as an electrocardiogram, that are indicative of a glucose level of the person 101. In the case of optical sensors, as persons skilled in the art will understand, an optical sensor may be configured to respond to the interaction or binding of a biological marker to a substrate by generating an electrical signal based on change in luminance of the substrate. For example, the substrate may include a material selected to fluoresce in response to contact with a selected biomarker, such as glucose. The fluorescence may be proportional to a concentration of the selected biomarker.

In some embodiments, the monitoring device 104 may include other types of sensors that may be worn, carried, or coupled to the person 101 to measure activity of the person 101 that may influence the glucose levels or glycemic response of the person 101. As an example, the sensors may include an acceleration sensor configured to detect an acceleration of the person 101 or a portion of the person 101, such as the person's hands or feet. The acceleration (or lack thereof) may be indicative of exercise, sleep, or food/beverage consumption activity of the person 101, which may influence the glycemic response of the person 101. In some embodiments, the sensors may include heart rate and/or body temperature, which may indicate an amount of physical exertion experienced by the person 101. In some embodiments, the sensors may include a GPS receiver which detects GPS signals to determine a location of the person 101.

The sensors described above are merely provided as examples. Other sensors or types of sensors for monitoring physiological condition, activity, and/or location, among other things, will be recognized by persons skilled in the art and are contemplated to be within the scope of the present disclosure. For any sensor, the signal provided by a sensor shall be referred to as a "sensor signal."

The monitoring device 104 may include components and/or circuitry configured to pre-process sensor signals. Pre-processing may include, without limitation, amplification, filtering, attenuation, scaling, isolation, normalization, transformation, sampling, and/or analog-to-digital conversion, among other things. Persons skilled in the art will recognize various implementations for such pre-processing, including, without limitation, implementations using processors, controllers, ASICS, integrated circuits, hardware, firmware, programmable logic devices, and/or machine-executable instructions, among others. The types of pre-processing and their implementations are merely provided as examples. Other types of pre-processing and implementations are contemplated to be within the scope of the present disclosure. In some embodiments, the monitoring device 104 may not perform pre-processing.

As used herein, the term "sensed data" shall mean and include the information represented by a sensor signal or by a pre-processed sensor signal. In some embodiments, sensed data may include glucose levels in a person 101, acceleration of a part of the person 101, heart rate of the person 101, temperature of the person 101, and/or geolocation (e.g., GPS location) of the person 101, among other things. The monitoring device 104 may communicate sensed data to the delivery device 102 via communication link 112 and/or to the computing device 106 via communication link 114. Use of sensed data by the delivery device 102 and/or by the computing device 106 will be described later herein.

The computing device 106 provides processing capabilities and may be implemented in various ways. In some embodiments, the computing device 106 may be a consumer device, such as a smartphone, a computerized wearable device (e.g., a smartwatch), a tablet computer, a laptop computer, or a desktop computer, among others, or may be a special purpose device (e.g., a portable control device) provided by, for example, the manufacturer of the delivery device 102. In some embodiments, the computing device 106 may be "processing circuitry" (defined below) that is integrated with another device, such as the delivery device 102. In some embodiments, the computing device 106 may be secured to the person 101 (e.g., to the body or clothing of person 101), may be at least partially implanted into the body of person 101, and/or may be held by the person 101.

For each of the embodiments of the computing device 106, the computing device 106 may include various types of logic circuitry, including, but not limited to, microprocessors, controllers, digital signal processors (DSPs), application specific integrated circuits (ASICs), field-programmable gate arrays (FPGAs), central processing units (CPU), graphics processing units (GPU), programmable logic devices, memory (e.g., random access memory, volatile memory, non-volatile memory, etc.), or other discrete or integrated logic circuitry, as well as combinations of such components. The term "processing circuitry" may generally refer to any of the foregoing logic circuitry, alone or in combination with other logic circuitry, or any other circuitry for performing computations.

Aspects of the delivery device 102, the monitoring device 104, and the computing device 106 have been described above. One or more of the devices 102-106 may include a user interface (not shown) that presents information to the person 101 and/or receives information from the person 101. The user interface may include a graphical user interface (GUI), a display device, a keyboard, a touchscreen, a speaker, a microphone, a vibration motor, buttons, switches, and/or other types of user interfaces. Persons skilled in the art will recognize various types of user interfaces that may be used, and all such user interfaces are contemplated to be within the scope of the present disclosure. For example, where the computing device 106 is a consumer device such as a smart phone, tablet computer, laptop computer, or the like, the user interfaces would include a display device, a physical and/or virtual keyboard, and/or audio speakers provided by such consumer devices, among other things. In some embodiments, a user interface may notify the person 101 of sensed data (e.g., glucose level) and/or insulin delivery data (e.g., rates of historic, current, or future insulin delivery) and may present alerts to the person 101. In some embodiments, a user interface may receive inputs from the person 101, which may include, for example, a requested change in insulin delivery and/or a meal indication, among other things. The descriptions and embodiments above regarding user interfaces are merely provided as examples, and other types and other uses of user interfaces are contemplated to be within the scope of the present disclosure.

The following describes communications between the devices 102-106 and cooperation between the devices 102-106 with respect to insulin delivery. As depicted in FIG. 1, and as mentioned above, the devices 102-106 may communicate with each other via communication links 112-116. In some embodiments, the computing device 106 may control operation of the delivery device 102 and/or the monitoring device 104. For example, the computing device 106 may generate one or more signals (e.g., a command signal) that cause the delivery device 102 to deliver insulin to the person 101, e.g., as a basal dosage and/or a bolus dosage. In some embodiments, the computing device 106 may receive data associated with insulin delivery (e.g., insulin delivery data) from the delivery device 102 and/or receive sensed data (e.g., glucose levels) from the monitoring device 104 and may perform computations based on the insulin delivery data, the sensed data, and/or other data to control the delivery device 102. Insulin delivery data may include, but is not limited to, a type of insulin being delivered, historical insulin delivery rates and/or amounts, current insulin delivery rate and/or amount, and/or user input affecting insulin delivery. As persons skilled in the art will understand, in a closed-loop operating mode, computing device 106 may communicate dosage commands to the delivery device 102 based on a difference between a current glucose level in the body of the person 101 (e.g., received from the monitoring device 104) and a target glucose level (e.g., determined by the computing device 106). The dosage commands may indicate an amount of insulin to be delivered and/or a rate of insulin delivery and may regulate the current glucose level toward the target glucose level. Examples of closed-loop operations for insulin infusion systems are described in United States Patent Nos.: 6,088,608, 6,119,028, 6,589,229, 6,740,072, 6,827,702, 7,323,142, and 7,402,153, and in United States Patent Application Publication Nos.: 2014/0066887 and 2014/0066889. The entire contents of each of the foregoing patents and publications are hereby incorporated by reference herein.

With continuing reference to FIG. 1, the remote or cloud computing system 108 may be a proprietary remote/cloud computing system or a commercial cloud computing system including one or more server computing devices. The remote/cloud computing system 108 may provide additional computing resources on-demand as needed when the computing resources of a client computing device (e.g., the computing device 106) are not sufficient. The computing device 106 and the remote/cloud computing system 108 may communicate with each other through a communication link 118, which may traverse one or more communication networks (not shown). The communication networks may include, without limitation, an Ethernet network, Wi-Fi network, a cellular network, a satellite network, an intranet, an extranet, the Internet, and/or the Internet backbone, among other types of networks. Persons skilled in the art will recognize implementations for the remote/cloud computing system 108 and how to interface with such systems through various types of networks. For example, the remote/cloud computing system 108 may include an array of processing circuitry (defined above) and may execute machine-readable instructions. Such implementations, interfaces, and networks are contemplated to be within the scope of the present disclosure. In some embodiments, the delivery device 102 and/or the monitoring device 104 may communicate directly with the remote or cloud computing system 108 via one or more other communication links (not shown in FIG. 1).

An example therapy delivery system has been described above. For convenience, the description below may primarily refer to an insulin delivery system as an example of the therapy delivery system. However, it is intended that any aspect, embodiment, or description relating to an insulin delivery system shall be applicable to a therapy delivery system which delivers a therapy other than insulin.

### Machine Learning Models

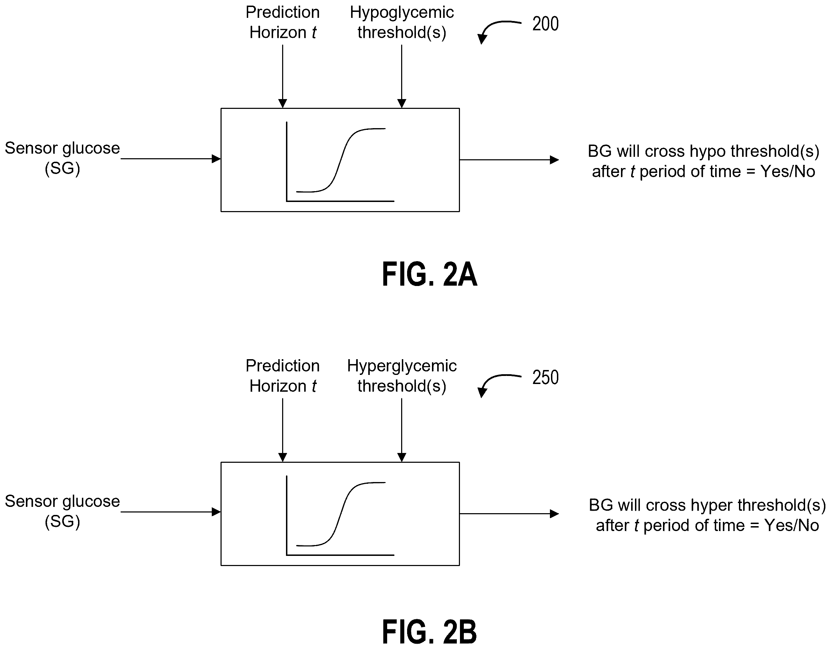

FIGS. 2A and 2B illustrate example machine learning (ML) models 200, 250 that are trained to predict a future physiological parameter (e.g., blood glucose value) after a period of time, according to some embodiments. In some embodiments, each of the ML models 200, 250 may be configured to receive as input: (i) one or more measured sensor glucose (SG) values, which may be in the form of glucose concentration values, (ii) a prediction horizon, which refers to how far ahead a ML model predicts the future, represented by some amount of time (e.g., 30 minutes), and/or (iii) one or more prediction thresholds. An example of the prediction horizon is 60 minutes, a fixed period of time. In some implementations, the prediction horizon may be changed to a different time period after a ML model has been trained, e.g., based on the training methodology noted below.

In some embodiments, each of the ML models 200, 250 may be a classifier configured to, based on the inputs and the parameter(s) of the ML model, generate as output a discrete prediction (e.g., yes or no, 1 or 0) as to whether a user's blood glucose (BG) will cross each of the one or more thresholds after a predetermined period of time. Some examples of the ML models 200, 250 may be based at least on, e.g., gradient boosting, decision trees, random forests. In some implementations, the probability or likelihood associated with the prediction may be generated and outputted. Such predetermined period of time may be dictated by the prediction horizon, referring to how far ahead a prediction is made. As examples of BG thresholds, a patient may be considered to be hypoglycemic if BG has fallen below a threshold of 70 mg/dL and hyperglycemic if BG has risen above a threshold of 120 mg/dL. However, these are merely examples illustrating a difference between a hypoglycemic threshold and a hyperglycemic threshold. Myriad thresholds may be considered and configured as input to ML models 200, 250, such as, for example, any value (inclusive) between 60-80 mg/dL for a low "floor" threshold to determine hypoglycemia, wherein a BG value (or multiple sustained BG values) crossing or being detected as below the low threshold would be indicative of hypoglycemia (or at least be the basis for such an indication); and any value (inclusive) between 120-400 mg/dL for a high threshold to determine hyperglycemia, wherein a BG value (or multiple sustained BG values) crossing or being detected as above the high threshold would be indicative of hyperglycemia (or at least be the basis for such an indication). It will be noted that low and high thresholds that overlap with euglycemic ranges (such as 80-120 mg/dL) may be used in some cases, such as 60-90 mg/dL for a low threshold and 100-400 mg/dL for a high threshold. Moreover, in some embodiments, multiple thresholds may be set. BG values crossing some of these thresholds may not indicate hypoglycemia or hyperglycemia. However, the ML models 200, 250 observing one or more of multiple thresholds being crossed may provide flags or other additional data, e.g., to determine a trend. If using a classifier as noted above, the prediction may be a binary determination (yes or no) for each of the one or more thresholds. Thus, at least one of the ML models 200, 250 may be a binary and multi-class model that is configured to make binary determinations with respect to multiple classes (e.g., thresholds).

The parameter(s) of each ML model may be, for example, weight(s) and/or bias(es) determined through training via loss minimization (e.g., gradient descent) after feeding a training dataset. In some cases, the ML models may each be population-trained, where the training dataset is obtained from a population of patient. For example, the training dataset may include BG values over time of numerous patients, including at one or more fixed time periods (30 minutes after a time of prediction, 60 minutes after a time of prediction, etc.), and may also include labeled data (e.g., whether a given BG value does or does not cross a threshold) and a validation dataset (e.g., 20% of the training data). The ML model may be trained with different specific time periods, but the ML model may be able to make predictions for other time periods as well, e.g., based on training on corresponding data across time periods. In some cases, the ML models may each be patient-specific, where the training dataset is obtained from patient information. For example, the training dataset may include BG values over time of a specific patient or user, including at one or more fixed time periods and may also include labeled data (e.g., whether a given BG value does or does not cross a threshold) and a validation dataset (e.g., 20% of the training data). A resulting ML model may be a classifier configured to predict, e.g., based on a logistic regression model, whether a given BG value will cross a given threshold. In some embodiments, a first ML model may be configured to determine whether a BG value will be below a threshold only (hypoglycemia), while a separate, second ML model may be trained to determine whether a BG value will be above a threshold only (hyperglycemia). In some implementations, a probability associated with the determination may also be considered, where, for example, an output of "yes" or "no" may be produced only if the probability of a BG value is above or below 50% or another probability threshold.

Thus, each of the ML models 200, 250 may be configured to predict the BG value and whether the BG value of the patient will cross a particular threshold (or thresholds) within a fixed period of time into the future (timeframe t corresponding to the prediction horizon). As an illustrative example, ML model 200 may be trained to determine future hypoglycemic conditions by determining whether, after some predetermined period of time (e.g., prediction horizon of one hour), a user's BG will be lower than a chosen floor, e.g., 70 mg/dL. More than one threshold may be provided, e.g., 80 mg/dL, 70 mg/dL and 60 mg/dL. In some implementations, the threshold(s) may be set by the user. In some implementations, preset threshold settings may be provided to the user, e.g., as a default or preconfigured setting. In some implementations, threshold settings may be provided and limited to a range, e.g., 60-90 mg/dL, such that a user cannot set a threshold above or below the range. However, in some implementations, other thresholds such as those that are typically considered non-hypoglycemic or non-hyperglycemic may be set (e.g., 90 mg/dL).

To illustrate with an example, the ML model 200 may first estimate the user's BG at the end of the predetermined period of time (prediction horizon). For example, ML model 200 may determine that BG will be 65 mg/dL after one hour. If thresholds of 70 mg/dL and 60 mg/dL have been set, for example, the determined BG would fall below the threshold of 70 mg/dL but not the threshold of 60 mg/dL. The ML model 200 may thus produce a prediction that the user's BG will cross the hypoglycemic threshold of 70 mg/dL but not the hypoglycemic threshold of 60 mg/dL. If the hypoglycemic low threshold was set to 70 mg/dL, ML model 200 may produce a determination and/or alert that the threshold has been reached. If the threshold was set to 60 mg/dL, ML model 200 may produce a determination that the threshold has not been reached. In configurations in which alerts or notifications are generated upon detection of the BG crossing a threshold, no alert may be generated, or an alert indicating that the threshold has not been reached may be generated.

Similarly, ML model 250 may be trained separately (e.g., from ML model 200) to determine future hyperglycemic conditions by determining whether, after some predetermined period of time (e.g., prediction horizon of one hour), a user's BG will be higher than a chosen ceiling threshold, e.g., 100 mg/dL. More than one threshold may be provided. The threshold(s) may be set by the user, or preset threshold settings may be provided to the user. Threshold settings may be limited to a permitted range, e.g., 100-400 mg/dL, such that a user cannot set a threshold above or below the range. The ML model 250 may first estimate the user's BG after the predetermined period of time. For example, ML model 250 may determine that BG will be 125 mg/dL after one hour. If thresholds of 120 mg/dL and 130 mg/dL have been set, the determined BG would be above the threshold of 120 mg/dL but not the threshold of 130 mg/dL. The ML model 250 may thus produce a prediction that the user's BG will cross the hyperglycemic threshold of 120 mg/dL but not the hyperglycemic threshold of 130 mg/dL.

As will be discussed further below, a second prediction horizon may be obtained, e.g., from the user or preselected values. In some embodiments, this second prediction horizon may be selected to be an amount of time that is less than the predetermined period of time (first, initial prediction horizon discussed above) used by a ML model to make the initial ML-based prediction. In one example, the second prediction horizon may be 30 minutes, which is less than the one hour from the above examples. In some embodiments, the second prediction horizon may be used at least in part to make a prediction at the initial prediction horizon.

### Predictive System

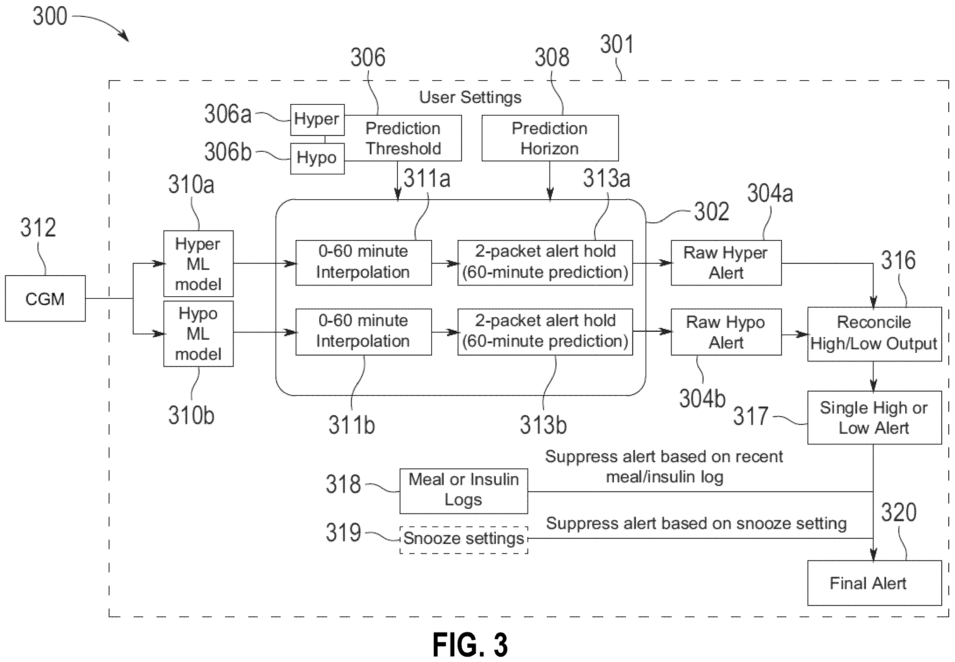

FIG. 3 is a diagram of an example architecture 300 having a predictive system 302, according to some embodiments. In some embodiments, the example architecture 300 includes the predictive system 302, which may be configured to output a raw indication (e.g., a raw hyperglycemia indication 304a and/or a raw hypoglycemia indication 304b). In some implementations, the predictive system 302 may be implemented on the delivery device 102, the monitoring device 104, or on the computing device 106. In some cases, the raw indication may refer to an indication generated by the predictive system 302 but not delivered to the user unless it meets one or more additional criteria before generating a final indication 320 to the user. However, in some cases, the raw indication may be passed directly to the user as the final indication 320 without further consideration of the additional criteria.

In some embodiments, the predictive system 302 may obtain as input one or more prediction thresholds 306 and one or more prediction horizons 308. The predictive system 302 may obtain one or more prediction thresholds for hyperglycemia 306a and one or more prediction thresholds for hypoglycemia 306b. In some embodiments, the prediction thresholds 306 and the prediction horizons 308 may be provided by the user. In some implementations, however, prediction thresholds 306 and prediction horizons 308 may be preconfigured by the predictive system 302, which in some cases may be default values that are adjustable by the user. In some cases, multiple prediction thresholds may be provided or preconfigured.

In some embodiments, a raw indication generated by the predictive system 302 may be a raw hyperglycemia indication 304a that indicates that the user will have a blood glucose level that is higher than one or more prediction thresholds 306a within a prediction horizon 308, or a raw hypoglycemia indication 304b that indicates that the user will have a blood glucose level that is lower than a prediction threshold 306b within the prediction horizon 308.

In some embodiments, the predictive system 302 may obtain inputs from one or more machine learning (ML) models. In the example architecture 300, the predictive system 302 may obtain inputs from a hyperglycemic ML model 310a configured to determine future hyperglycemic conditions based on a fixed period of time into the future, and a hypoglycemic ML model 310b configured to determine future hypoglycemic conditions based on a fixed period of time into the future. The fixed period of time may be a time horizon that is pre-selected or pre-determined based on a configuration of the hyperglycemic ML model 310a and/or the hypoglycemic ML model 310b. In some cases, the fixed period of time for the hyperglycemic ML model 310a may be different from the fixed period of time for the hypoglycemic ML model 310b. For example, the fixed period of time may be 60 minutes for both models in some cases; however, in some cases, it may be 60 minutes for one model and 45 minutes for another model. Hyperglycemic ML model 310a may be an example of the ML model 250, and hypoglycemic ML model 310b may be an example of the ML model 200. Hence, in some embodiments, each of the ML models 310a, 310b may be trained or may have been trained as discussed above with respect to FIGS. 2A and 2B, and may be embodied as hardware, firmware, logic, machine-executable instructions, and/or controlled by processors, controllers, ASICS, integrated circuits, programmable logic devices, other hardware, other firmware, and/or other machine-executable instructions. In some implementations, the ML models 310a, 310b may be pre-trained and obtained from the remote or cloud computing system 108, or they may be preconfigured and stored on the delivery device 102, the monitoring device 104, or the computing device 106.

Each of the ML models 310a, 310b may obtain, as input, sensor glucose (SG) readings measured from a monitoring device 312. In some embodiments, the monitoring device 312 may include a continuous glucose monitor (CGM), which may include one or more sensors configured to estimate blood glucose based on interstitial glucose of a user. Monitoring device 312 may be an example of the monitoring device 104 in some implementations. In some embodiments, as discussed above with respect to FIGS. 2A and 2B, ML models 310a, 310b may include classifiers configured to output a prediction as to whether the blood glucose (BG) level of the user will cross a particular threshold (or thresholds) within a fixed period of time into the future, e.g., 60 minutes.

In specific implementations where the predictive system 302 is implemented on the computing device 106, the computing device 106 can receive SG readings and measurements as inputs (e.g., discrete inputs or a continuous input stream), where the inputs can be from the CGM (e.g., monitoring device 312). In some cases, inputs may also be received from delivery device 102. Some or all other parts of predictive system components 301 may also be implemented on the computing device 106 along with the predictive system 302.

In some embodiments, as part of a secondary prediction output, ML model 310a or 310b may first predict the BG value of the user at the end of the fixed period of time, according to a selected or predetermined horizon, e.g., 60 minutes. Then, the predictive system 302 may be configured to use the ML-predicted BG value for the period of time to determine or estimate the BG value for a different prediction horizon that is less than the fixed period of time provided above. The different prediction horizon may be provided as a prediction horizon 308, which may be selectable or provided by a user or patient, or configured as a default or otherwise determined by the example architecture 300 or the predictive system 302. This determination or estimation of the BG value may be based on an analytical technique that uses the ML-predicted BG value and an anchor SG value (obtained via the monitoring device 312). An "anchor" SG value may refer to the SG value at the time of prediction. In some implementations, the analytical technique may include interpolation(s) 311a and/or 311b, such as linear interpolation between the ML-predicted BG value and the anchor SG value. In other implementations, alternative types of interpolation may be used, including exponential, logarithmic, power, polynomial, cubic, etc.

As an example of linear interpolation, a line may be fitted between the two values. If a prediction horizon 308 is less than the fixed period of time used to predict the BG value (e.g., the prediction horizon 308 is 30 minutes as compared to the 60-minute fixed period of time for the ML-based prediction by a ML model, e.g., 310a or 310b), an intermediate value may be determined for the 30-minute prediction horizon. The prediction horizon for the intermediate value may be referred to as a "target prediction horizon" that is different from the fixed period of time used for the ML-based prediction, which may also be referred to as an "anchor prediction horizon" for the purposes of interpolation.

Interpolation may be beneficial in cases where the prediction horizon(s) of interest are less suitable for ML-based prediction of BG values, or cases where multiple predictions are needed or where predictions need to be made for different or quickly changing prediction horizons. It may also be useful for reducing usage of computational resources. To illustrate, for instance, a user may estimate a BG value using ML models once (or just a few times) given an anchor prediction horizon, e.g., 60 minutes. From this BG value, an interpolation can be performed quickly and/or in quick succession based on a current SG reading (e.g., anchor SG value 412) and one or more chosen target prediction horizons. In fact, multiple interpolations can be performed substantially at once to provide estimated BG values for multiple target prediction horizons that are less than the anchor prediction horizon, e.g., at 10 minutes, 20 minutes, 30 minutes, 40 minutes, and 50 minutes. As opposed to using interpolation, using ML models to generate each of these predictions of BG values may be more time- and resource-consuming. More granular and exact estimations of BG values can be made as well if desired (e.g., using a target prediction horizon of 31 minutes).

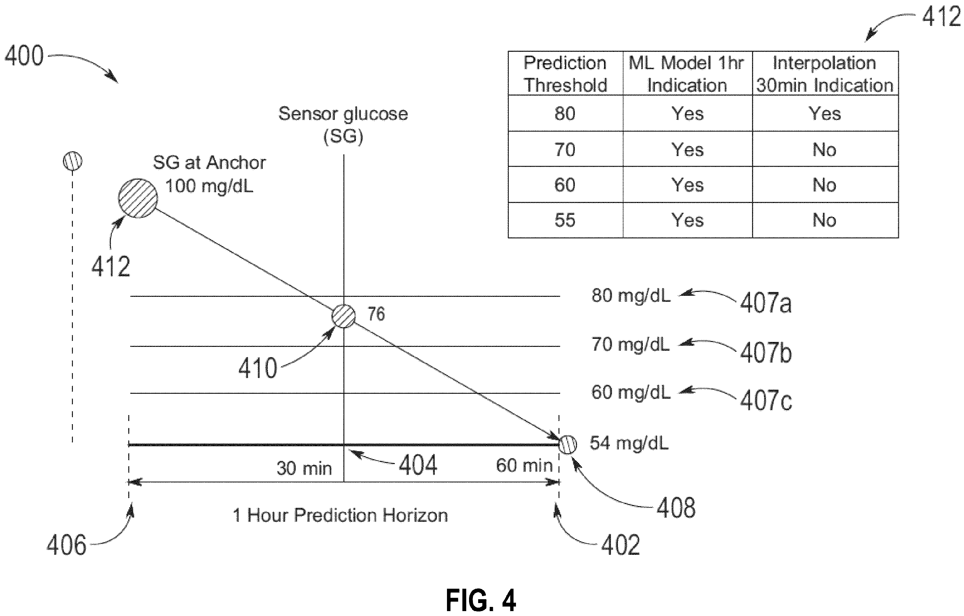

Referring to FIG. 4 to illustrate an example graph 400 illustrating interpolation to determine a potential hypoglycemic indication (e.g., hypoglycemic alert), the anchor prediction horizon used by the ML model to predict the BG value may be 60 minutes (time 402), as was the case in examples discussed previously with respect to FIGS. 2A and 2B. The target prediction horizon may be less than the anchor prediction horizon, 30 minutes in this example (time 404). The target prediction horizon may be selected from other ranges of time (e.g., by the user or preconfigured or preselected by the predictive system 302), such as between 1 and 59 minutes, or any time period less than the anchor prediction horizon (e.g., a 75-minute target prediction horizon may be selected if the anchor prediction horizon is 90 or 120 minutes). These example time periods are with respect to an "anchor time," a current measurement or reference time. This means that the time period between an anchor time (e.g., anchor time 406) and the time 402 is 60 minutes, and the time period between the anchor time and the time 404 is 30 minutes.

In this case, an ML-predicted BG value 408 of 54 mg/dL based on the anchor prediction horizon may be considered the lowest SG value. Since the anchor SG value 412 at an anchor time 406 is higher (100 mg/dL) than the ML-predicted BG value 408 of 54 mg/dL, the intermediate value will logically fall between the lowest SG value of 54 mg/dL and the anchor value of 100 mg/dL. Given the target prediction horizon of 30 minutes as an input, the predictive system 302 will predict a final SG value 410 of 76 mg/dL, which as shown in FIG. 4 at time 404 is between the ML-predicted BG value 408 (at time 402) and the anchor SG value 412 (at the anchor time 406). While other, longer target or anchor prediction horizons may be used, such as 120 minutes, 180 minutes, etc., longer prediction horizons may be less accurate in certain situations, or they may make it more difficult for a user to act on it or remember to do so without aid (e.g., alarm or reminder) because, e.g., future circumstances such as intervening activities (e.g., meal, insulin delivery, snack, exercise, sleep) or the availability of a caretaker or the user may become more difficult to predict the farther out the time horizon. Thus, the use scenario, usability, and usefulness of the predictions may be considered when selecting the anchor and target prediction horizons.

In some approaches, taking a line fitted between the ML-predicted BG value at time 402 and at least the anchor SG value 412 (at anchor time 406), the predictive system 302 may determine that a final predicted BG value 410 is 76 mg/dL for the target prediction horizon at time 404, as shown in FIG. 4. As alluded to elsewhere herein, SG (sensor glucose) may refer to sensor-estimated BG (blood glucose), e.g., based on interstitial glucose. As shown on the example graph 400, there may be multiple prediction thresholds 407a-407c such as 80, 70 and 60 mg/dL. The prediction thresholds may be obtained from the user (e.g., via input means) or predetermined, below which the predictive system 302 may generate a raw hypoglycemic indication. Hence, if the prediction threshold is selected to be 80 mg/dL, for example, the predictive system 302 may generate a raw hypoglycemic indication for the prediction horizon of 30 minutes, since the final predicted BG value 410 of 76 mg/dL is below the prediction threshold of 80 mg/dL. In other words, to illustrate with an example procedure based on FIG. 4, an ML model such as the hypoglycemic ML model 310b may first generate a predicted BG value of 54 mg/dL according to the anchor prediction horizon of 60 minutes (using, e.g., techniques described with respect to FIG. 2A), and then, based on the predicted BG value, the predictive system 302 may generate a final predicted BG value of 76 mg/dL according to the target prediction horizon of 30 minutes (using, e.g., analytical technique such as interpolation 311b), and finally, the predictive system 302 may generate (or not) a raw hypoglycemic indication 304b based on whether the final predicted BG value of 76 mg/dL is below a prediction threshold 306b (which may be one of prediction thresholds 407a-407c). In some cases, the hypoglycemic ML model 310b may determine a probability or likelihood of accuracy of the ML-predicted BG value 408 and only output the final predicted BG value 410 and whether it will cross a given threshold (e.g., 306b, e.g., 407a) if the probability of crossing the given threshold is over a probability threshold (e.g., at least 90%, 80%, 50%, etc.). In some cases, e.g., where the chosen prediction horizon 308 is the same as the fixed time period for the ML-based prediction based on the anchor prediction horizon, a raw hypoglycemic indication for the prediction threshold of 60 minutes may be generated. That is, no interpolation may be needed if the anchor prediction horizon is the same as the target prediction horizon.

On the other hand, if the prediction threshold 306 is selected by the user or otherwise set to 70, 60 or 55 mg/dL, the raw hypoglycemic indication for the target prediction threshold of 30 minutes may not be triggered since final predicted SG value of 76 mg/dL is above each of these thresholds. However, in some cases, a raw hypoglycemic indication for the anchor (or target) prediction threshold of 60 minutes may be generated since the ML-predicted BG is below the threshold of 55 mg/dL. The thresholds and indications that may be generated based on respective prediction horizons are depicted by a table 412 shown in FIG. 4.

In some implementations, the comparison of the interpolated SG value and the threshold may not be so exact. The final predicted BG value 410 may have a range associated with it, such as an uncertainty, probability, or error bar or other indication (e.g., heatmap or distribution of probabilities). If the range associated with the final predicted SG value 410 overlaps a threshold such that the upper end of the range is over a threshold and the lower end of the range is under a threshold, one of various types of indications or alerts may result. For instance, a secondary indication or alert may be generated where it does not indicate to users that their BG will cross the threshold but indicates its potential or a probability of crossing the threshold (e.g., a user may see a yellow light instead of an orange light depending on the probability, or similar progressive or graded indication or alert). As another example, the predictive system 302 may temporarily increase its target prediction horizon (e.g., to 40 minutes increased from 30 minutes) and provide a raw indication if the predictive system 302 determines that the final predicted BG value 410 and at least a majority of its range is under the threshold (e.g., after 40 minutes rather than 30 minutes, most of the error bar associated with the final predicted BG value 410 is under the threshold). In another approach, the predictive system 302 may use the overlap for later consideration. As noted above and will be discussed below, the example architecture 300 may consider one or more additional criteria before generating and providing a final indication 320 to the user. Examples of providing the final indication 320 to the user may include, but are not limited to, providing a visual, haptic, and/or audio alert to the user (e.g., at one or more devices such as a delivery device 102, a monitoring device 104, a computing device 106, and/or a separate display or user interface associated with the user), providing a visual, haptic, and/or audio alert to a caretaker (e.g., on similar devices such as the foregoing one or more devices), providing a visual, haptic, and/or audio alert to a remote location (e.g., server or cloud computing system 108). In some cases, the generated final indication 320 may be stored but not provided (e.g., as an alert). Such cases may occur based on, e.g., settings, contextual information 318, and/or user preference or settings 319 as will be described further below.

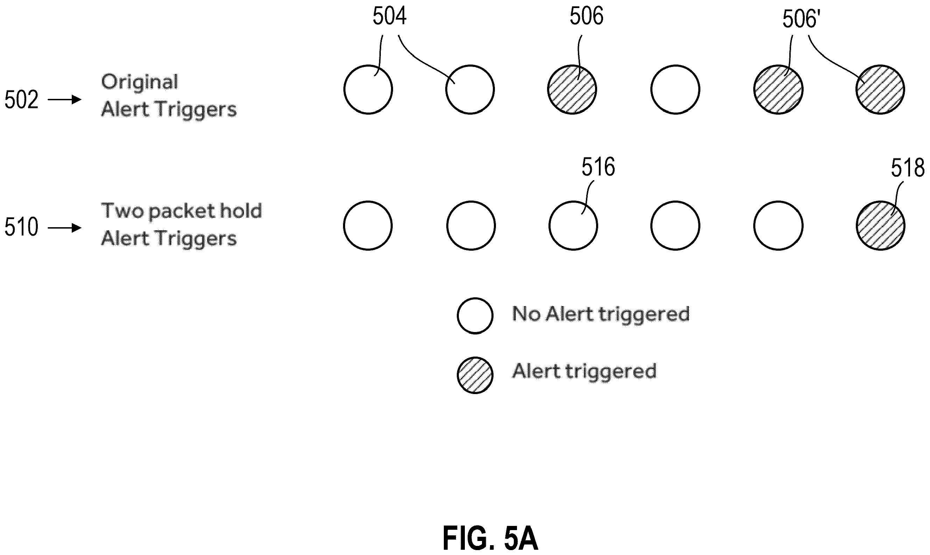

In some implementations, the error range for the final predicted BG value 410 may be another such additional criterion before a final indication 320 is produced. As another example, and as will be further discussed below with respect to FIGS. 5A - 5C, the overlap may be used as an indication or an alert in a "hold" approach (e.g., if the range overlaps the threshold but the predicted BG value 410 is below the threshold), or a "partial" alert (e.g., if the range overlaps the threshold but the predicted BG value 410 is above the threshold).

In other embodiments, a corresponding curve (e.g., exponential curve, logarithmic curve) may be fitted for other types of interpolation, and a raw indication may be generated based on the selected threshold(s) as with the linear interpolation as shown in FIG. 4.

A similar approach to the above may be taken for generating a raw hyperglycemic indication 304a. More specifically, the hyperglycemic ML model 310a may predict a BG value of the user at the end of the fixed period of time according to an anchor prediction horizon. For example, (i) the hyperglycemic ML model 310a may be configured to determine a BG value of 120 mg/dL according to the anchor prediction horizon (with an associated probability or likelihood in some implementations), (ii) the predictive system 302 may be configured to determine a final predicted BG value based on the target prediction horizon (e.g., using an analytical technique such as interpolation 311a), and (iii) the predictive system 302 may be configured to determine whether the final predicted BG value will cross above a given threshold (e.g., at least one of one or more prediction thresholds for hyperglycemia 306a). Depending on the determination of whether or not the predicted BG value will cross one or more selected prediction thresholds 306a for the selected prediction horizon 308, the predictive system 302 may output a raw hyperglycemia indication 304a. In some cases, the predicted BG value may have a probability or error range associated with it, which may be used in various ways before generating the final indication 320, as described above.

In some implementations, the predictive system 302 may "hold" a prediction of hyperglycemia (at block 313a) or a prediction of hypoglycemia (at block 313b) that would otherwise be output from the predictive system 302 as a raw hyperglycemia indication 304a or a raw hypoglycemia indication 304b. In various approaches, certain conditions may be required before the "held" prediction(s) are delivered as a raw hyperglycemia or hypoglycemia indication 304a or 304b. Using this approach, sensitivity of generating a prediction may be maintained or adjusted to an appropriate or desired extent, and false positives may advantageously be reduced depending on the configuration. FIGS. 5A - 5C illustrate specific applications of this "hold" approach.

Referring to FIG. 5A, two rows are shown representing predictions by a ML model, where the predictions would otherwise result in (or "trigger") a raw hyperglycemia or hypoglycemia indication if they were not held for further validation according to conditions. In some implementations, these predictions may be generated using the predictive system 302. In some approaches, interpolation 311a or 311b as discussed above may be implemented as part of generating the predictions. However, in some implementations, the predictions may be generated by the hyperglycemic ML model 310a or the hypoglycemic ML model 310b as discussed above, and provided directly to block 313a or 313b.

The approach in the FIG. 5A example may be one in which two consecutive triggering predictions are a condition for the predictive system 302 (via block 313a or 313b) to produce a raw hyperglycemic indication 304a or hypoglycemic indication 304b. Put differently, two preliminary predictions (or "packets") are held to deliver a raw indication 304a or 304b. As illustrated in FIG. 5A, the top row 502 includes two non-triggering predictions 504 (represented by empty circles) and a triggering prediction 506 (represented by a solid circle). In implementations that do not hold predictions, the triggering prediction 506 may result in a raw hyperglycemic or hypoglycemic indication as expected, as discussed elsewhere herein, e.g., with respect to FIG. 3 or 4. However, it can be seen in the bottom row 510 that, if a prediction is held until a condition is met, the raw indication may not be triggered by the corresponding prediction 516. Since two consecutive triggering predictions are needed to meet the condition, a raw indication 518 is generated and released from block 313a or 313b once there are two triggering predictions 506' and the latest one of the predictions is qualified for release. As noted elsewhere herein, the predictions may be generated at intervals such as every 5 minutes. Hence, at 5-minute intervals, at least 10 minutes would be required to validate the predictions and generate the raw indication 518. The raw indication 518 is produced on the second triggering prediction, which in some implementations may be in the form of an alert delivered to a user. As also noted, this holding approach can lower the sensitivity of the predictions and reduce false positives, which may be adjusted and configured as desired. Below is another approach that could further reduce sensitivity and false positives.

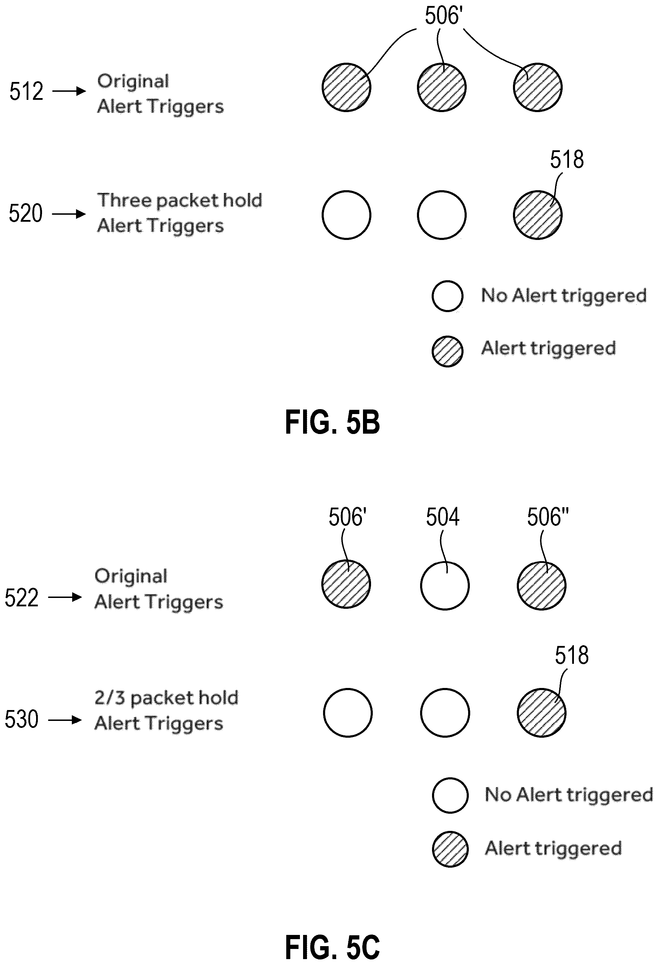

Turning now to FIG. 5B, a three-packet hold, which is a variant of the two-packet hold discussed with respect to FIG. 5A, is illustrated. Here, the condition to produce the raw indication may be three consecutive triggering predictions. The top row 512 shows three triggering predictions 506' output from the predictive system 302 (e.g., via interpolation 311a or 311b), or from the hyperglycemic ML model 310a or the hypoglycemic ML model 310b, occurring consecutively. Since this meets the established criterion for this approach using a three-packet hold, the raw indication 518 is produced from block 313a or 313b on the third triggering prediction. As one having ordinary skill in the relevant arts may appreciate, other similar conditions may be set, such as four or more consecutive triggering predictions. However, one will also appreciate that overly strict conditions may reduce the efficacy of providing a reliable indication of a hypoglycemia or hyperglycemia to the user.

FIG. 5C is another variant of the hold approach in which two triggering predictions within three previous predictions is a condition. Unlike previous variants, this does not require consecutive triggering predictions. As shown in the top row 522, a triggering prediction 506' occurs first, a non-triggering prediction 506 occurs next, and a second triggering prediction 506" occurs afterward. A raw indication 518 is generated on the second triggering prediction 506", as it meets the "2/3-packet hold" condition. This approach validates the predictions to a more relaxed extent compared to the previous approaches, as it requires two triggering prediction within given boundaries (e.g., three predictions). One can appreciate myriad variants to this approach, such as two out of four previous predictions, three out of four previous predictions, three out of five previous predictions, and so on, depending on the desired sensitivity.

In some embodiments, the holding of the predictions as described above may be an optional condition to evaluate at blocks 313a and 313b for producing the raw hyperglycemic indication 304a or the raw hypoglycemic indication 304b, respectively.

In some scenarios, the example architecture 300 may optionally reconcile (at block 316) a raw hyperglycemic indication 304a and a raw hypoglycemic indication 304b that are obtained simultaneously or substantially simultaneously (e.g., within a prescribed time of each other such as 3 seconds). One or more of various types of rules may be imposed for the reconciliation. Depending on the low and high prediction thresholds selected by the user and/or depending on the prediction horizons, there could be instances when the predictive system 302 may predict and trigger a raw hyperglycemic indication 304a and a raw hypoglycemic indication 304b simultaneously. Simultaneous raw hyperglycemic and hypoglycemic indications may result from, e.g., prediction thresholds for hyperglycemia and hypoglycemia being too similar, overlapping error ranges, too short a target prediction horizon (not allowing much change between the anchor SG value 412 and the ML-predicted BG value 408), or model errors. To reconcile such a result, in some configurations, the preference may be to suppress the raw hyperglycemic indication 304b and allow the raw hypoglycemic indication 304a to proceed and pass as a single raw indication 317. In some configurations, the preference may be to suppress the raw hypoglycemic indication 304a and allow the raw hyperglycemic indication 304b to proceed. The preference may be pre-selected or provided depending on the desired sensitivity level. For example, it may be known that a user is susceptible to hypoglycemia, in which case, the predictive system 302 may be configured to prefer the raw hypoglycemic indication 304a if simultaneous indications ever result. In some implementations, both the raw hypoglycemic indication 304a and the raw hyperglycemic indication 304b may be suppressed. Since conflicting raw indications may indicate that the raw indications may not be reliable or immediately useful to the patient monitoring (e.g., it may be indicative of a euglycemic condition), no alert may be generated if both raw indications are suppressed. In some implementations, however, both the raw hypoglycemic indication 304a and the raw hyperglycemic indication 304b may be allowed. In such a case, a user may manually evaluate both raw indications to determine the next action. Alternatively, in some cases, the example architecture 300 may use past historical patterns associated with the raw indications, probabilities or likelihoods associated with the raw indications, user activities (meal, insulin log, exercise, etc.) within a recent time period such that they are unlikely to significantly affect predictions, or other data to determine and/or generate a likely outcome (whether the raw hypoglycemic indication 304a is valid or the raw hyperglycemic indication 304b is valid) at block 316. As mentioned above, however, this reconciliation is optional and may not be performed, e.g., when simultaneous raw indications do not occur.

In some embodiments, the foregoing techniques to generate the final indication 320 may be performed at a regular interval (e.g., every 5 minutes according to prediction thresholds 306 and prediction horizon 308). In some embodiments, the final indication 320 may be generated on an as-needed basis (e.g., user request with prediction thresholds 306 and prediction horizon 308 provided by user or used according to existing configuration).

However, there may be exceptions to the foregoing generation of the final indication 320. In some implementations, final indications 320 (e.g., in the form of alerts to the user) may be suppressed at least temporarily based on contextual information 318 and/or user preference or settings 319.

For example, if contextual information 318 or event such as a meal or an insulin log is identified within a period of time (e.g., 15 minutes of the anchor time 406), then the final indication 320 (e.g., an alert to the user) may not be triggered for at least one upcoming interval (or indefinitely, e.g., until the next raw indication), as the user may be intervening to avoid an excursion and fluctuations in BG, thus obviating unnecessary alerts. In some situations, exercise or other physical activity by a user or ingestion of medication or other substances by a user may be detected (e.g., using a physiological monitor or sensor). In such cases, the final indication 320 may not be triggered for at least one upcoming interval (or indefinitely), as physiological changes (e.g., higher metabolism or energy consumption) may affect the BG of the user. In some contexts, if a variation in anchor SG that is more than a certain amount or percentage is detected within the above period of time (e.g., 15 minutes), the final indication 320 may not be triggered.

As another example of an exception, the final indication 320 or other alerts may be suppressed depending on one or more settings 319 by the user, such as a snooze setting that temporarily or indefinitely (e.g., until enabled again) prevents or disables indications or alerts from being given to the user, or an alert schedule that regularly allows alerts during certain times and/or prevents alerts during certain times. In some implementations, any indication or alert that would have been provided may be discarded if there is a snooze in effect, or saved and provided after the snooze setting is disabled or has expired.

Putting together and summarizing the various approaches and techniques described above, in one illustrative example scenario, a predictive system 302 may receive inputs including: one or more prediction thresholds 306 including one or more prediction thresholds for hyperglycemia 306a and one or more prediction thresholds for hypoglycemia 306b, and one or more prediction horizons 308. Two (or more) ML models (e.g., a hyperglycemic ML model 310a and a hypoglycemic ML model 310b) may receive SG readings from a monitoring device 312 (a CGM being one example). Based on the SG readings and the output of the two (or more) ML models (e.g., ML-predicted BG values at the end of a fixed period of time, or binary predictions of crossing a threshold within the fixed period of time), the predictive system 302 may output and provide a prediction as to as to whether the BG level of a user will cross one or more selected prediction thresholds 306 over a selected prediction horizon 308. If the prediction of the predictive system 302 is a hyperglycemic prediction that the BG will be over a prediction threshold, then a raw hyperglycemic indicator 304a may be generated. If the prediction of the predictive system 302 is a hypoglycemic prediction that the BG will be below a prediction threshold, then a raw hypoglycemic indicator 304b may be generated. The raw indications may be binary (yes or no). In some implementations, binary raw indications may be generated for each of the one or more prediction thresholds 306. Assuming there are no conflicting predictions to reconcile (at block 316), no uncertainty overlap, and no exceptions such as contextual information 318 (e.g., meal, insulin log, activity that may result in excursion) or user settings 319, a final indication 320 may be generated, e.g., in the form of an alert or notification to the user.

In some implementations, a prediction of euglycemia (normal concentration of blood glucose) may be generated and/or provided, e.g., if neither a prediction of hyperglycemia nor hypoglycemia is made, although this may simply be assumed in an absence of the hyperglycemia or hypoglycemia prediction, indication, or alert to the user.

In another illustrative example scenario, the predictive system 302 may receive an anchor prediction horizon and/or a target prediction horizon (among the one or more prediction horizons 308). For instance, the anchor prediction horizon may be 60 minutes, and the target prediction horizon may be shorter, e.g., 30 minutes. The predictive system 302 may perform an analytical technique (e.g., interpolation 311a or 311b) such as interpolation (linear, exponential, etc., or a combination thereof) based on the anchor prediction horizon to predict a final SG value 410. The raw indication 304a or 304b at the target prediction horizon may depend on whether the final SG value 410 is below or above one or more prediction thresholds 306. In some implementations, binary raw indications may be generated for each of the one or more prediction thresholds 306. The anchor prediction horizon may be the same as the fixed period of time used to generate the ML-predicted BG values (e.g., 60 minutes). However, the fixed period of time and consequently the anchor prediction horizon may be changed for future predictions by the ML models 310a, 310b and/or by the predictive system 302.

In another illustrative example scenario, two or more preliminary predictions may be held to evaluate whether they meet a condition (e.g., two or three triggering predictions) before releasing a qualifying prediction as raw indication 304a or 304b (e.g., at block 313a or 313b). This way, predictions may need further validation and confirmation before being provided to the user (which can, e.g., reduce false positives).

As noted above, the predictive system 302 and/or other parts of predictive system components 301 can be implemented on various devices, such as the delivery device 102, the monitoring device 104, or on the computing device 106. For example, in implementations on the computing device 106, the computing device 106 could be a mobile device (e.g., smartphone) that can receive inputs (e.g., from monitoring device 312 and/or elsewhere) such as SG measurements and perform aforementioned functionalities, such as (but not limited to) performing analytical techniques (e.g., interpolation 311a or 31 1b), holding predictions (block 313a or 313b), reconciling conflicting predictions (block 316), evaluating contextual information or user settings (block 318 or 319), and generating a final indication (320) and/or providing an alert or notification to the user.

In some implementations, the example architecture 300 may be implemented (e.g., as an App) on a user device (e.g., a smartphone) and may be used for multiple daily injection (MDI) therapy, which involves multiple (e.g., three or more) deliveries of insulin per day to a patient. For instance, the user device may receive inputs from monitoring device 312 and generate the final indication 320 to alert the patient or a caretaker to a prediction of future high sensor glucose events so that bolus insulin (e.g., rapid acting or short acting) can advantageously be administered proactively and flexibly as needed throughout the day (e.g., overnight, in between meals) rather than rigidly with fixed doses and a fixed schedule. Similarly, a user can be alerted to a prediction of future low sensor glucose events to counteract with carbohydrates.

### Example Methods

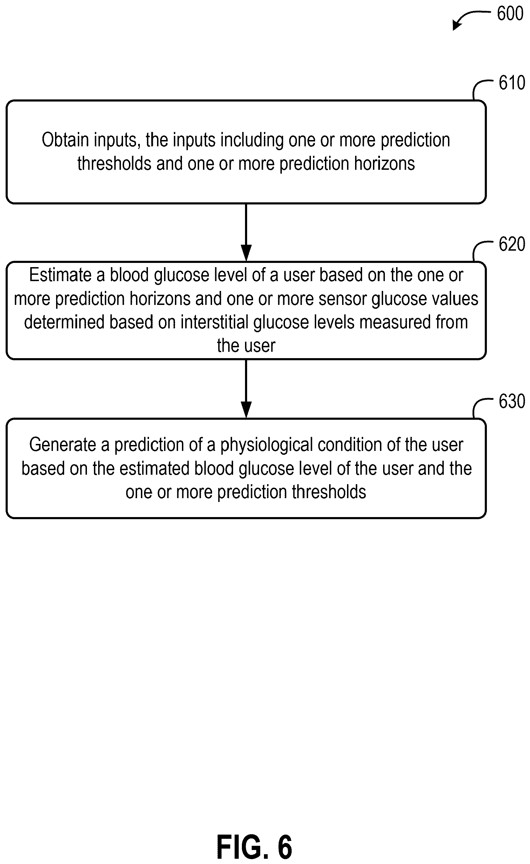

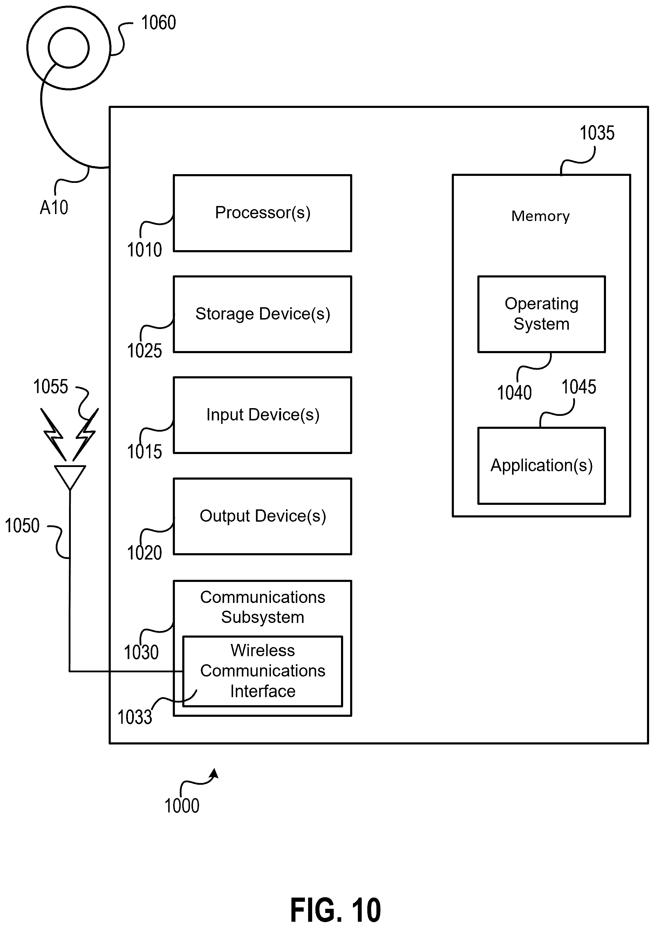

FIG. 6 is a flow diagram of an example of a method 600 for predicting a physiological condition of a user, according to one or more embodiments. The method 600 may be performed by one or more devices (e.g., device 102, device 104, device 106, and/or system 108). Each of the one or more devices may include hardware and/or software components, such as, for example, one or more processors (e.g., a microcontroller and/or a microprocessor) and/or one or more processor-readable media storing instructions which, when executed by one or more processors, cause performance of one or more operations. Example components one or more devices are illustrated in FIG. 10, which are described in more detail below. It should also be noted that the method 600 may be performed in any suitable order, not necessarily the order depicted in FIG. 6. Further, the method 600 may include additional or fewer operations than those depicted in FIG. 6.

At block 610, the method 600 may include obtaining inputs, the inputs including one or more prediction thresholds and one or more prediction horizons.

At block 620, the method 600 may include estimating a blood glucose level of the user based on the one or more prediction horizons and one or more sensor glucose values determined based on interstitial glucose levels measured from the user.

At block 630, the method 600 may include generating a prediction of the physiological condition of the user based on the estimated blood glucose level of the user and the one or more prediction thresholds. In some embodiments, an indication of the prediction may be provided to the user, e.g., via a display or user interface associated with the user, e.g., at one or more devices such as a delivery device 102, a monitoring device 104, and/or a computing device 106. In some cases, the alert may be provided elsewhere (e.g., a location remote to the user, such as a server, remote or cloud computing system 108, or a caretaker's location) or stored for later analysis or provision (e.g., if alerts are snoozed).

In some embodiments, the prediction may be a binary output (e.g., yes or no, 1 or 0), and the physiological condition of the user may be hyperglycemia or hypoglycemia. In some cases, a prediction of euglycemia may be generated and/or provided, e.g., if neither a prediction of hyperglycemia nor hypoglycemia is made, although this may simply be assumed in an absence of the hyperglycemia or hypoglycemia prediction, indication, or alert to the user.

As discussed above with respect to FIGS. 3 - 5C, the prediction may be generated and/or qualified based on various one or more factors.

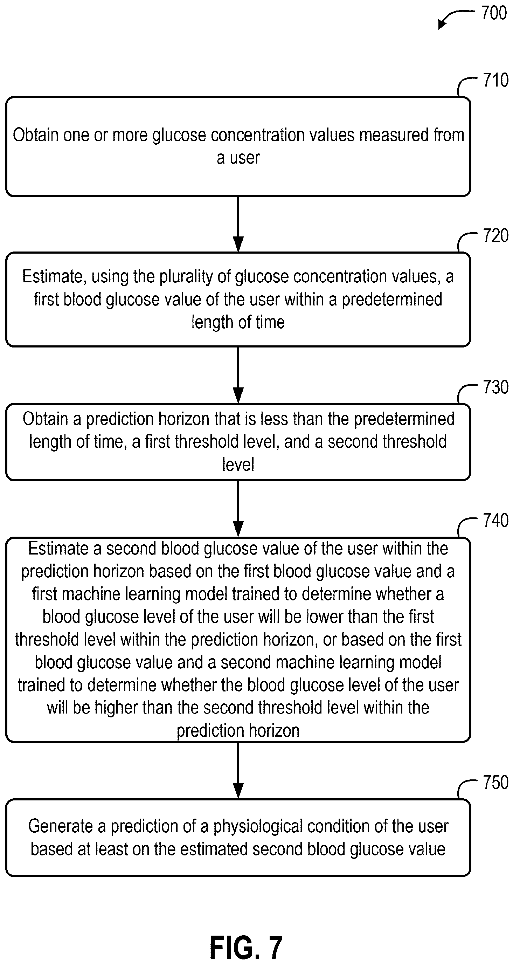

FIG. 7 is a flow diagram of another example of a method 700 for predicting a physiological condition of a user, according to one or more embodiments. The method 700 may be performed by one or more devices (e.g., device 102, device 104, device 106, and/or system 108). Each of the one or more devices may include hardware and/or software components, such as, for example, one or more processors (e.g., a microcontroller and/or a microprocessor) and/or one or more processor-readable media storing instructions which, when executed by one or more processors, cause performance of one or more operations. Example components one or more devices are illustrated in FIG. 10, which are described in more detail below. It should also be noted that the method 700 may be performed in any suitable order, not necessarily the order depicted in FIG. 7. Further, the method 700 may include additional or fewer operations than those depicted in FIG. 7.

At block 710, the method 700 may include obtaining one or more glucose concentration values measured from the user. In some embodiments, the glucose concentration values may include interstitial glucose levels of the user measured by a sensor. The interstitial glucose levels may be the basis for determining sensor glucose (SG) values referred to herein.

At block 720, the method 700 may include, estimating, using the plurality of glucose concentration values, a first blood glucose value of the user within a predetermined length of time. In some embodiments, the predetermined length of time may be relative to when the first blood glucose value is estimated. In some embodiments, the estimating of the first blood glucose value of the user within the predetermined length of time may be performed at a prescribed interval (e.g., every 5 minutes). In some embodiments, the estimating of the first blood glucose value of the user within the predetermined length of time may include estimating the first blood glucose value of the user using a first trained machine learning model or a second trained machine learning model; the first trained machine learning model may be trained to predict whether a future blood glucose value of the user will be lower than one or more first thresholds of the prediction thresholds within the predetermined length of time; and the second trained machine learning model may be trained to predict whether the future blood glucose value of the user will exceed one or more second thresholds of the prediction thresholds within the predetermined length of time. In some embodiments, the first and second trained machine learning models may each include a classifier trained to at least determine a probability of the future blood glucose of the user breaching the one or more first thresholds or the one or more second thresholds within the predetermined length of time

At block 730, the method 700 may include obtaining a prediction horizon that is less than the predetermined length of time, a first threshold level, and a second threshold level. In some embodiments, the predetermined length of time may be 60 minutes, and the prediction horizon may be under 60 minutes (or other time period less than the predetermined length of time). It will be recognized that various other timeframes may be selected, as discussed above.

At block 740, the method 700 may include estimating a second blood glucose value of the user within the prediction horizon based on the first blood glucose value and a first machine learning model trained to determine whether a blood glucose level of the user will be lower than the first threshold level within the prediction horizon, or based on the first blood glucose value and a second machine learning model trained to determine whether the blood glucose level of the user will be higher than the second threshold level within the prediction horizon. In some implementations, the first ML model may be an example of ML model 200, and the second ML model may be an example of ML model 250. In some implementations, the method may further include, based on the second blood glucose value being below the first threshold level or above the second threshold level, generating an indication that the second blood glucose value will breach the first threshold level or the second threshold level within the prediction horizon. In some implementations, the method may further include obtaining the prediction horizon, the first threshold level, and the second threshold level from the user. In some embodiments, the estimating of the second blood glucose value of the user within the prediction horizon may include an interpolation using the first glucose value and the at least one glucose concentration value of the one or more glucose concentration values. In some implementations, the interpolation may include a linear interpolation between the first glucose value and the at least one glucose concentration value.

At block 750, the method 700 may include generating a prediction of the physiological condition of the user based at least on the estimated second blood glucose value. For example, in some scenarios, the estimated second blood glucose value may be lower than the first threshold level, which may indicate, for example, hypoglycemia. In some scenarios, the estimated second blood glucose value may be higher than the second threshold level, which may indicate, for example, hyperglycemia. In some cases, a binary determination may be generated to indicate a physiological condition (e.g., hypoglycemia is yes/no or hyperglycemia is yes/no).

In some embodiments, the method may further include providing an alert to the user, the alert indicative of the generated prediction of the physiological condition. For example, the alert may be provided on a display or user interface associated with the user, e.g., at one or more devices such as a delivery device 102, a monitoring device 104, and/or a computing device 106. In some cases, the alert may be provided elsewhere (e.g., a location remote to the user, such as a server, remote or cloud computing system 108, or a caretaker's location) or stored for later analysis or provision (e.g., if alerts are snoozed). However, in some embodiments, the method may further include generating an alert, the alert indicative of the generated prediction of the physiological condition; and suppressing the alert to prevent delivery of the alert to the user based on a contextual event, a user setting, or a combination thereof.

In some embodiments, the generating of the prediction of the physiological condition of the user may include generating a plurality of preliminary predictions based on the based on the estimated second blood glucose value and the one or more prediction thresholds, and determining that the plurality of preliminary predictions meet a condition. In some implementations, the condition may include the plurality of preliminary predictions being triggered consecutively, or at least a portion of the plurality of preliminary predictions being triggered, causing the raw indication of the prediction to be "held" in the meantime. In some embodiments, the generating of the prediction of the physiological condition of the user may include generating a first prediction relating to a first physiological condition and second prediction a second physiological condition, and reconciling the first and second predictions based on a rule (e.g., based on block 316).

In some cases, the physiological condition of the user may be hyperglycemia, while in some other cases, the physiological condition of the user may be hypoglycemia. In some embodiments, the one or more prediction thresholds may include a plurality of prediction thresholds, and generating the prediction of the physiological condition of the user may include generating a respective prediction of the physiological condition of the user for each of the plurality of prediction thresholds.

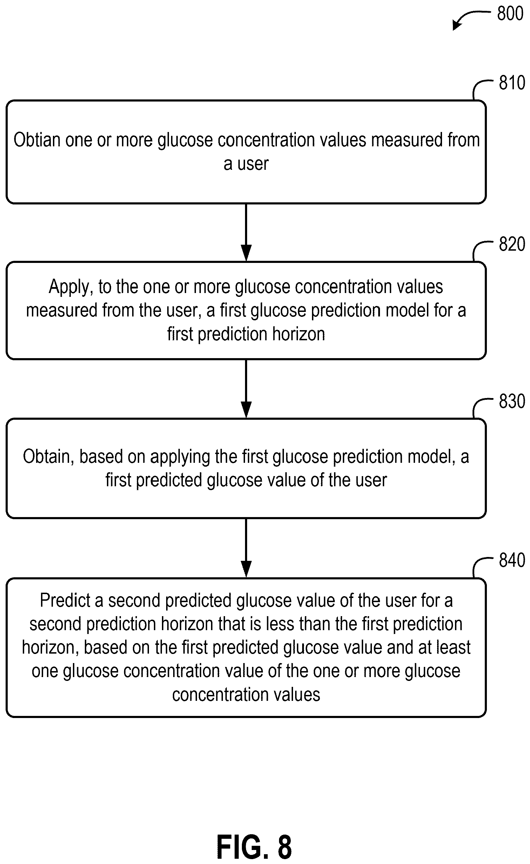

FIG. 8 is a flow diagram of another example of a method 800 for predicting a physiological condition of a user, according to one or more embodiments. The method 800 may be performed by one or more devices (e.g., device 102, device 104, device 106, and/or system 108). Each of the one or more devices may include hardware and/or software components, such as, for example, one or more processors (e.g., a microcontroller and/or a microprocessor) and/or one or more processor-readable media storing instructions which, when executed by one or more processors, cause performance of one or more operations. Example components one or more devices are illustrated in FIG. 10, which are described in more detail below. It should also be noted that the method 800 may be performed in any suitable order, not necessarily the order depicted in FIG. 8. Further, the method 800 may include additional or fewer operations than those depicted in FIG. 8.

At block 810, the method 800 may include obtaining one or more glucose concentration values measured from a user. In some embodiments, the one or more glucose concentration values may be obtained based on interstitial glucose levels of the user measured with a sensor device.

At block 820, the method 800 may include applying, to the one or more glucose concentration values measured from the user, a first glucose prediction model for a first prediction horizon.

At block 830, the method 800 may include obtaining, based on applying the first glucose prediction model, a first predicted glucose value of the user. In some embodiments, the first prediction horizon may be relative to when the first predicted glucose value is obtained. In some embodiments, the obtaining of the first predicted glucose value of the user may be performed at a prescribed interval.

In some embodiments, the obtaining of the first predicted glucose value of the user may include estimating the first predicted glucose value of the user using the first glucose prediction model and a second glucose prediction model; one of the first glucose prediction model or the second glucose prediction model may be trained to predict whether a future blood glucose value of the user will be lower than one or more first thresholds within the first prediction horizon; and another one of the first glucose prediction model or the second glucose prediction model may be trained to predict whether the future blood glucose value of the user will exceed one or more second thresholds within the first prediction horizon. In some implementations, the first and second glucose prediction models may each include a classifier trained to at least determine a probability of the future blood glucose value of the user breaching the one or more first thresholds or the one or more second thresholds within the first prediction horizon.

At block 840, the method 800 may include predicting a second predicted glucose value of the user for a second prediction horizon that is less than the first prediction horizon, based on the first predicted glucose value and at least one glucose concentration value of the one or more glucose concentration values. In some embodiments, the first prediction horizon may be 60 minutes, and the second prediction horizon may be under 60 minutes. In some embodiments, the predicting of the second glucose value of the user within the prediction horizon may include an interpolation using the first predicted glucose value and the at least one glucose concentration value of the one or more glucose concentration values. In some implementations, the interpolation may include a linear interpolation between the first predicted glucose value and the at least one glucose concentration value.

In some embodiments, the method 800 may further include generating, based on the second predicted glucose value breaching a predetermined threshold level, a notification that the predetermined threshold level is predicted to be breached within the second prediction horizon. In some implementations, the method 800 may further include suppressing the notification to prevent delivery of the notification to the user based on a contextual event, a user setting, or a combination thereof.

In some embodiments, the method 800 may further include obtaining a first prediction threshold or a second prediction threshold; and generating a prediction of a physiological condition of the user based on the predicted second glucose value and the first prediction threshold or the second prediction threshold. In some implementations, the second prediction horizon, the first prediction threshold, and the second prediction threshold may be obtained from the user.

In some embodiments, the method 800 may further include generating a prediction of a physiological condition of the user, the generating of the prediction comprising generating a plurality of preliminary predictions based on the predicted second glucose value and one or more prediction thresholds, and determining that the plurality of preliminary predictions meet a condition. In some implementations, the condition may include the plurality of preliminary predictions being triggered consecutively, or at least a portion of the plurality of preliminary predictions being triggered.

In some embodiments, the method 800 may further include generating a prediction of a physiological condition of the user, the generating of the prediction comprising generating a first prediction relating to a first physiological condition and second prediction a second physiological condition, and reconciling the first and second predictions based on a rule (e.g., based on block 316).

### Example Apparatus

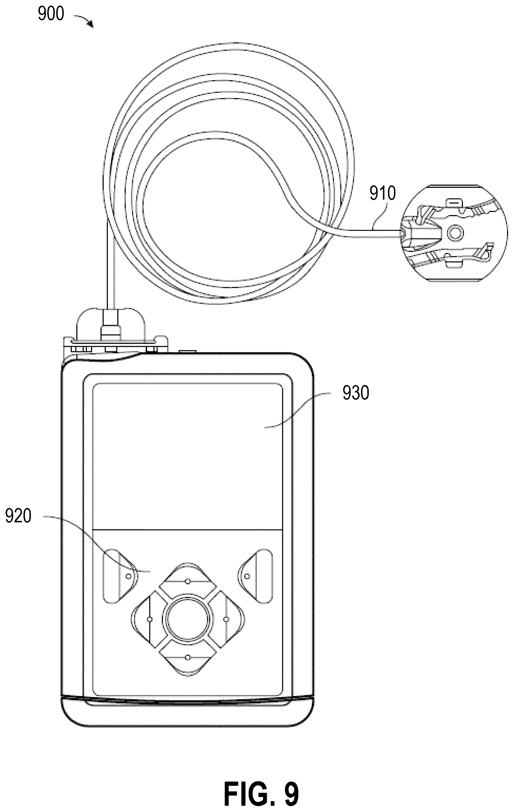

In some embodiments, therapy may be effected based on communicating a therapy determination toward a therapy delivery device. A non-limiting example of such a device is described below in connection with FIG. 9.

As mentioned above, therapy determinations may be communicated toward an insulin delivery device 900 (e.g., from a cloud computing system 108 via an intermediary computing device 106 communicatively coupled to the device 90). In such a device, insulin delivery may be performed based on internal communication between a central computing module (e.g., a microcontroller for device 900 as a whole) and an insulin delivery module (e.g., including a microcontroller, a motor, and a pump). For instance, insulin delivery may be caused by the central computing module communicating a delivery command in the form of an electrical signal that travels via a communication fabric to the insulin delivery module. The central computing module may also be configured to communicate (e.g., via a transceiver) with a computing device (e.g., 106, FIG. 1) communicatively coupled to a remote or cloud computing system (e.g., 108, FIG. 1). The insulin delivery device 900 may communicate various event data toward the remote or cloud computing system, which may communicate insulin delivery determinations toward the insulin delivery device 900, in accordance with the techniques described herein.

The insulin delivery device 900 can provide fast-acting insulin through a small tube 910 configured for fluidic connection with a cannula inserted subcutaneously. The device 900 can deliver two types of dosages-a basal dosage, which can be delivered periodically (e.g., every five minutes) in tiny amounts throughout the day and night, and a bolus dosage to cover an increase in blood glucose from meals and/or to correct high blood glucose levels. The depicted insulin delivery device 900 includes a user interface having button elements 920 that can be manipulated to administer a bolus of insulin, to change therapy settings, to change user preferences, to select display features, and the like. The insulin delivery device 900 also includes a display device 930 that can be used to present various types of information or data to the user. In accordance with aspects of the present disclosure, a user of the insulin delivery device 1200 may use the button elements 920 to input certain event data (e.g., event type, event start time, event details, etc.), and the user inputs can be confirmed using the display device 930. The depicted insulin delivery device 900 of FIG. 9 is merely provided by way of example, and other types of insulin delivery devices and other techniques different from those described above are contemplated to be within the scope of the present disclosure.

FIG. 10 is a block diagram of an embodiment of a computer system 1000, which can be utilized in embodiments as described herein. It should be noted that FIG. 10 is meant only to provide a generalized illustration of various components, any or all of which may be utilized as appropriate. In addition, it can be noted that components illustrated by FIG. 10 can be localized to a single device (e.g., delivery device 102, monitoring device 104, computing device 106, or computing system 108) and/or distributed among various networked devices, which may be disposed at different geographical locations.

In some embodiments, the computer system 1000 may include hardware elements that can be electrically coupled via a bus (or may otherwise be in communication, as appropriate). The hardware elements may include processor(s) 1010, which may comprise, without limitation, one or more microcontroller(s), one or more microprocessor(s), one or more general-purpose processors, one or more special-purpose processors (such as digital signal processing chips, graphics acceleration processors, and/or the like), and/or other processing structure, which can be configured to perform one or more of the methods or functionalities described herein.

In some embodiments, the computer system 1000 also may include one or more input devices 1015, which may comprise, without limitation, button elements 920, a microphone, a glucose sensor, and/or the like. The computer system 1000 may also include one or more output devices 1020, which may comprise without limitation a display device (e.g., 930), a speaker, a buzzer, and/or the like.

In some embodiments, the computer system 1000 may further include one or more non-transitory storage devices 1025, which can include, without limitation, local and/or network accessible storage, and/or may comprise, without limitation, a disk drive, a drive array, an optical storage device, a solid-state storage device, such as a read-access memory (RAM) and/or read-only memory (ROM), which can be programmable, flash-updateable, and/or the like. Such storage devices may be configured to implement any appropriate data stores, including without limitation, various file systems, database structures, and/or the like. Such data stores may include database(s) and/or other data structures used to store and administer messages and/or other information to be sent to one or more other components or external devices.

In some embodiments, the computer system 1000 may also include a communications subsystem 1030, which may implement wireless communication technologies managed and controlled by a wireless communication interface 1033. Additionally or alternatively, communications subsystem 1030 may implement wired technologies (such as Ethernet, coaxial communications, universal serial bus (USB), or the like). The wireless communication interface 1033 may comprise one or more wireless transceivers that may send and receive wireless signals (e.g., signals according to Bluetooth^{®}, Bluetooth Low Energy (BLE)). Thus, the communications subsystem 1030 may comprise a modem, a network card (wireless or wired), an infrared communication device, a wireless communication device, and/or a chipset, and/or the like, which may enable the computer system 1000 to communicate with any device discussed with respect to FIG. 1, including delivery device 102, monitoring device 104, computing device 106, and/or a cloud computing system 108 as described herein. Hence, the communications subsystem 1030 may be used to receive and send data (e.g., SG, BG) as described in the embodiments herein.

In some embodiments, the computer system 1000 will further comprise a working memory 1035, which may comprise a RAM or ROM device, as described above. Software elements, shown as being located within the working memory 1035, may comprise computer-readable and computer-executable instructions 1040; device drivers; executable libraries; and/or other code, which may comprise computer programs used in various embodiments and/or may be designed to implement methods and/or configure systems in accordance with embodiments described herein. Merely by way of example, one or more operations described with respect to the methods or functionalities discussed above might be implemented as code and/or instructions executable by a computer (and/or a processor within a computer). Such code and/or instructions can be used to configure and/or adapt a general-purpose computer (or other device) to perform one or more operations in accordance with the described methods.

In some embodiments, a set of these instructions and/or code may be stored on a non-transitory computer-readable storage medium, such as the storage device(s) 1025 described above. In some cases, the storage medium might be incorporated within a computer system, such as computer system 1000. In other embodiments, the storage medium might be separate from a computer system (e.g., a removable medium, such as an optical disc) and/or provided in a downloadable installation package, such that the storage medium can be used to program, configure, and/or adapt a general purpose computer with the instructions and/or code stored thereon. These instructions might take the form of executable code, which is executable by the computer system 1000, and/or might take the form of source and/or installable code, which, upon compilation and/or installation on the computer system 1000 (e.g., using any of a variety of generally available compilers, installation programs, compression/decompression utilities, etc.), then takes the form of executable code.

The embodiments disclosed herein are examples of the disclosure and may be embodied in various forms. For instance, although certain embodiments herein are described as separate embodiments, each of the embodiments herein may be combined with one or more of the other embodiments herein. Specific structural and functional details disclosed herein are not to be interpreted as limiting, but as a basis for the claims and as a representative basis for teaching one skilled in the art to variously employ the present disclosure in virtually any appropriately detailed structure. Like reference numerals may refer to like elements throughout the description of the figures.

Any of the herein described techniques, operations, methods, programs, algorithms, or codes may be converted to, or expressed in, a programming language or computer program embodied on a computer, processor, or machine-readable medium. The terms "programming language" and "computer program," as used herein, each include any language used to specify instructions to a computer or processor, and include (but is not limited to) the following languages and their derivatives: Assembler, Basic, Batch files, BCPL, C, C+, C++, Delphi, Fortran, Java, JavaScript, machine code, operating system command languages, Pascal, Perl, PL1, Python, scripting languages, Visual Basic, metalanguages which themselves specify programs, and all first, second, third, fourth, fifth, or further generation computer languages. Also included are database and other data schemas, and any other meta-languages. No distinction is made between languages which are interpreted, compiled, or use both compiled and interpreted approaches. No distinction is made between compiled and source versions of a program. Thus, reference to a program, where the programming language could exist in more than one state (such as source, compiled, object, or linked) is a reference to any and all such states. Reference to a program may encompass the actual instructions and/or the intent of those instructions.

It should be understood that the foregoing description is only illustrative of the present disclosure. To the extent consistent, any or all of the aspects detailed herein may be used in conjunction with any or all of the other aspects detailed herein. Various alternatives and modifications can be devised by those skilled in the art without departing from the disclosure. Accordingly, the present disclosure is intended to embrace all such alternatives, modifications, and variances. The embodiments described with reference to the attached drawing figures are presented only to demonstrate certain examples of the disclosure. Other elements, steps, methods, and techniques that are insubstantially different from those described above and/or in the appended claims are also intended to be within the scope of the disclosure.

While several embodiments of the disclosure have been depicted in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope and spirit of the claims appended hereto.

Further disclosed herein is the subject-matter of the following clauses:
1. A processor-implemented method, the method comprising:
   obtaining one or more glucose concentration values measured from a user;
   applying, to the one or more glucose concentration values measured from the user, a first glucose prediction model for a first prediction horizon;
   obtaining, based on applying the first glucose prediction model, a first predicted glucose value of the user; and
   predicting a second predicted glucose value of the user for a second prediction horizon that is less than the first prediction horizon, based on the first predicted glucose value and at least one glucose concentration value of the one or more glucose concentration values.
2. The method of clause 1, further comprising generating, based on the second predicted glucose value breaching a predetermined threshold level, a notification that the predetermined threshold level is predicted to be breached within the second prediction horizon.
3. The method of clause 1 or 2, further comprising:
   suppressing the notification to prevent delivery of the notification to the user based on a contextual event, a user setting, or a combination thereof.
4. The method of clause 1 or of any of the clauses 1 to 3, further comprising:
   obtaining a first prediction threshold or a second prediction threshold; and
   generating a prediction of a physiological condition of the user based on the predicted second glucose value and the first prediction threshold or the second prediction threshold.
5. The method of clause 4 or of any of the clauses 1 to 4, wherein the second prediction horizon, the first prediction threshold, and the second prediction threshold are obtained from the user.
6. The method of clause 1 or of any of the clauses 1 to 5, wherein the first prediction horizon is relative to when the first predicted glucose value is obtained.
7. The method of clause 1 or of any of the clauses 1 to 6, wherein the obtaining of the first predicted glucose value of the user is performed at a prescribed interval.
8. The method of clause 1 or of any of the clauses 1 to 7, wherein:
   the obtaining of the first predicted glucose value of the user comprises estimating the first predicted glucose value of the user using the first glucose prediction model and a second glucose prediction model;
   one of the first glucose prediction model or the second glucose prediction model is trained to predict whether a future blood glucose value of the user will be lower than one or more first thresholds within the first prediction horizon; and
   another one of the first glucose prediction model or the second glucose prediction model is trained to predict whether the future blood glucose value of the user will exceed one or more second thresholds within the first prediction horizon.
9. The method of clause 8 or of any of the clauses 1 to 8, wherein the first and second glucose prediction models each comprise a classifier trained to at least determine a probability of the future blood glucose value of the user breaching the one or more first thresholds or the one or more second thresholds within the first prediction horizon.
10. The method of clause 1 or of any of the clauses 1 to 9, wherein the one or more glucose concentration values are obtained based on interstitial glucose levels of the user measured with a sensor device.
11. The method of clause 1 or of any of the clauses 1 to 10, wherein the predicting of the second glucose value of the user within the prediction horizon comprises an interpolation using the first predicted glucose value and the at least one glucose concentration value of the one or more glucose concentration values.
12. The method of clause 11 or of any of the clauses 1 to 11, wherein the interpolation comprises a linear interpolation between the first predicted glucose value and the at least one glucose concentration value.
13. The method of clause 1 or of any of the clauses 1 to 12, further comprising generating a prediction of a physiological condition of the user, the generating of the prediction comprising generating a plurality of preliminary predictions based on the predicted second glucose value and one or more prediction thresholds, and determining that the plurality of preliminary predictions meet a condition.
14. The method of clause 13 or of any of the clauses 1 to 13, wherein the condition comprises the plurality of preliminary predictions being triggered consecutively, or at least a portion of the plurality of preliminary predictions being triggered.
15. The method of clause 1 or of any of the clauses 1 to 14, further comprising generating a prediction of a physiological condition of the user, the generating of the prediction comprising generating a first prediction relating to a first physiological condition and second prediction a second physiological condition, and reconciling the first and second predictions based on a rule.
16. The method of clause 1 or of any of the clauses 1 to 15, wherein the first prediction horizon is 60 minutes, and the second prediction horizon is under 60 minutes.
17. A system comprising:
   one or more processors; and
   one or more processor-readable media storing instructions which, when executed by the one or more processors, cause performance of:
      obtaining one or more glucose concentration values measured from a user;
      estimating, using the one or more glucose concentration values, a first blood glucose value of the user within a predetermined length of time;
      obtaining a prediction horizon that is less than the predetermined length of time, a first threshold level, and a second threshold level;
      estimating a second blood glucose value of the user within the prediction horizon based on the first blood glucose value and a first machine learning model trained to determine whether a blood glucose level of the user will be lower than the first threshold level within the prediction horizon, or based on the first blood glucose value and a second machine learning model trained to determine whether the blood glucose level of the user will be higher than the second threshold level within the prediction horizon; and
      generating a prediction of a physiological condition of the user based at least on the estimated second blood glucose value.
18. The system of clause 17, wherein:
   the first machine learning model is configured to:
      output a first raw indication of the second blood glucose value of the user based at least on at least one of the one or more glucose concentration values, the first blood glucose value of the user, the first threshold level, the prediction horizon, or a combination thereof; and
      generate the prediction of the physiological condition of the user based on the first raw indication of the second blood glucose value of the user, wherein the physiological condition is hyperglycemia; and
   the second machine learning model is configured to:
      output a second raw indication of the second blood glucose value of the user based at least on the at least one of the one or more glucose concentration values, the first blood glucose value of the user, the second threshold level, the prediction horizon, or a combination thereof; and
      generate the prediction of the physiological condition of the user based on the second raw indication of the second blood glucose value of the user, wherein the physiological condition is hypoglycemia.
19. The system of clause 17 or 18, wherein:
   the first machine learning model is further configured to generate the first raw indication based at least on an interpolation between the at least one of the one or more glucose concentration values, and the first blood glucose value of the user;
   the second machine learning model is further configured to generate the second raw indication based at least on an interpolation between the at least one of the one or more glucose concentration values, and the first blood glucose value of the user;
   the one or more processor-readable media storing instructions which, when executed by the one or more processors, further cause performance of providing, to the user, the prediction of the physiological condition of the user.
20. One or more non-transitory processor-readable media storing instructions which, when executed by one or more processors, cause performance of:
   obtaining one or more glucose concentration values measured from a user;
   applying, to the one or more glucose concentration values measured from the user, a first glucose prediction model for a first prediction horizon;
   obtaining, based on applying the first glucose prediction model, a first predicted glucose value of the user; and
   predicting a second predicted glucose value of the user for a second prediction horizon that is less than the first prediction horizon, based on the first predicted glucose value and at least one glucose concentration value of the one or more glucose concentration values.

Further disclosed herein is the subject-matter of the following items:
1. A processor-implemented method of operating a therapy delivery system (100) to predict a physiological condition of a user (101):
   obtaining one or more glucose concentration values measured from a user (101);
   applying, to the one or more glucose concentration values measured from the user, a first glucose prediction model (3 10a, 3 10b) for a first prediction horizon (402);
   obtaining, based on applying the first glucose prediction model, a first predicted glucose value (408) of the user; and
   predicting a second predicted glucose value (410) of the user for a second prediction horizon (404) that is less than the first prediction horizon, based on the first predicted glucose value (408) and at least one glucose concentration value (412) of the one or more glucose concentration values.
2. The method of item 1, further comprising generating, based on the second predicted glucose value breaching a predetermined threshold level (407a-407c), a notification that the predetermined threshold level is predicted to be breached within the second prediction horizon.
3. The method of item 2, further comprising:
   suppressing the notification to prevent delivery of the notification to the user based on a contextual event, a user setting, or a combination thereof.
4. The method of item 1 or 2, further comprising:
   obtaining a first prediction threshold or a second prediction threshold; and
   generating a prediction of a physiological condition of the user based on the predicted second glucose value and the first prediction threshold or the second prediction threshold.
5. The method of item 1 or 2, wherein the obtaining of the first predicted glucose value of the user is performed at a prescribed interval.
6. The method of any of the preceding items, wherein:
   the obtaining of the first predicted glucose value of the user comprises estimating the first predicted glucose value of the user using the first glucose prediction model and a second glucose prediction model;
   one of the first glucose prediction model or the second glucose prediction model is trained to predict whether a future blood glucose value of the user will be lower than one or more first thresholds within the first prediction horizon; and
   another one of the first glucose prediction model or the second glucose prediction model is trained to predict whether the future blood glucose value of the user will exceed one or more second thresholds within the first prediction horizon.
7. The method of item 6, wherein the first and second glucose prediction models each comprise a classifier trained to at least determine a probability of the future blood glucose value of the user breaching the one or more first thresholds or the one or more second thresholds within the first prediction horizon.
8. The method of any of the preceding items, wherein the one or more glucose concentration values are obtained based on interstitial glucose levels of the user measured with a sensor device.
9. The method of any of the preceding items, wherein the predicting of the second glucose value of the user within the prediction horizon comprises an interpolation using the first predicted glucose value and the at least one glucose concentration value of the one or more glucose concentration values.
10. The method of any of the preceding items, further comprising generating a prediction of a physiological condition of the user, the generating of the prediction comprising generating a plurality of preliminary predictions based on the predicted second glucose value and one or more prediction thresholds, and determining that the plurality of preliminary predictions meet a condition.
11. The method of item 10, wherein the condition comprises the plurality of preliminary predictions being triggered consecutively, or at least a portion of the plurality of preliminary predictions being triggered.
12. The method of any of the preceding items, further comprising generating a prediction of a physiological condition of the user, the generating of the prediction comprising generating a first prediction relating to a first physiological condition and second prediction a second physiological condition, and reconciling the first and second predictions based on a rule.
13. A system comprising:
   a therapy delivery system (100) comprising a therapy delivery device (102), a monitoring device (104), and a computing device (106), and optionally, a remote computing device (108);
   one or more processors associated with the therapy delivery system; and
   one or more processor-readable media storing instructions which, when executed by the one or more processors, cause performance of:
      obtaining one or more glucose concentration values measured from a user;
      estimating, using the one or more glucose concentration values, a first blood glucose value of the user within a predetermined length of time;
      obtaining a prediction horizon that is less than the predetermined length of time, a first threshold level, and a second threshold level;
      estimating a second blood glucose value of the user within the prediction horizon based on the first blood glucose value and a first machine learning model trained to determine whether a blood glucose level of the user will be lower than the first threshold level within the prediction horizon, or based on the first blood glucose value and a second machine learning model trained to determine whether the blood glucose level of the user will be higher than the second threshold level within the prediction horizon; and
      generating a prediction of a physiological condition of the user based at least on the estimated second blood glucose value.
14. The system of item 13, wherein:
   the first machine learning model is configured to:
      output a first raw indication of the second blood glucose value of the user based at least on at least one of the one or more glucose concentration values, the first blood glucose value of the user, the first threshold level, the prediction horizon, or a combination thereof; and
      generate the prediction of the physiological condition of the user based on the first raw indication of the second blood glucose value of the user, wherein the physiological condition is hyperglycemia; and
      the second machine learning model is configured to:
   output a second raw indication of the second blood glucose value of the user based at least on the at least one of the one or more glucose concentration values, the first blood glucose value of the user, the second threshold level, the prediction horizon, or a combination thereof; and
   generate the prediction of the physiological condition of the user based on the second raw indication of the second blood glucose value of the user, wherein the physiological condition is hypoglycemia.
15. The system of item 14, wherein:
   the first machine learning model is further configured to generate the first raw indication based at least on an interpolation between the at least one of the one or more glucose concentration values, and the first blood glucose value of the user;
   the second machine learning model is further configured to generate the second raw indication based at least on an interpolation between the at least one of the one or more glucose concentration values, and the first blood glucose value of the user;
   the one or more processor-readable media storing instructions which, when executed by the one or more processors, further cause performance of providing, to the user, the prediction of the physiological condition of the user.

## Claims

1. A processor-implemented method, the method comprising:
obtaining one or more glucose concentration values measured from a user;
applying, to the one or more glucose concentration values measured from the user, a first glucose prediction model for a first prediction horizon;
obtaining, based on applying the first glucose prediction model, a first predicted glucose value of the user; and
predicting a second predicted glucose value of the user for a second prediction horizon that is less than the first prediction horizon, based on the first predicted glucose value and at least one glucose concentration value of the one or more glucose concentration values.

2. The method of Claim 1, further comprising generating, based on the second predicted glucose value breaching a predetermined threshold level, a notification that the predetermined threshold level is predicted to be breached within the second prediction horizon,
particularly
further comprising:
suppressing the notification to prevent delivery of the notification to the user based on a contextual event, a user setting, or a combination thereof.

3. The method of Claim 1 or 2, further comprising:
obtaining a first prediction threshold or a second prediction threshold; and
generating a prediction of a physiological condition of the user based on the predicted second glucose value and the first prediction threshold or the second prediction threshold, particularly
wherein the second prediction horizon, the first prediction threshold, and the second prediction threshold are obtained from the user.

4. The method of any of Claims 1 to 3, wherein the first prediction horizon is relative to when the first predicted glucose value is obtained.

5. The method of any of Claims 1 to 4, wherein the obtaining of the first predicted glucose value of the user is performed at a prescribed interval.

6. The method of any of Claims 1 to 5, wherein:
the obtaining of the first predicted glucose value of the user comprises estimating the first predicted glucose value of the user using the first glucose prediction model and a second glucose prediction model;
one of the first glucose prediction model or the second glucose prediction model is trained to predict whether a future blood glucose value of the user will be lower than one or more first thresholds within the first prediction horizon; and
another one of the first glucose prediction model or the second glucose prediction model is trained to predict whether the future blood glucose value of the user will exceed one or more second thresholds within the first prediction horizon,
particularly
wherein the first and second glucose prediction models each comprise a classifier trained to at least determine a probability of the future blood glucose value of the user breaching the one or more first thresholds or the one or more second thresholds within the first prediction horizon.

7. The method of any of Claims 1 to 6, wherein the one or more glucose concentration values are obtained based on interstitial glucose levels of the user measured with a sensor device.

8. The method of any of Claims 1 to 7, wherein the predicting of the second glucose value of the user within the prediction horizon comprises an interpolation using the first predicted glucose value and the at least one glucose concentration value of the one or more glucose concentration values,
particularly
wherein the interpolation comprises a linear interpolation between the first predicted glucose value and the at least one glucose concentration value.

9. The method of any of Claims 1 to 8, further comprising generating a prediction of a physiological condition of the user, the generating of the prediction comprising generating a plurality of preliminary predictions based on the predicted second glucose value and one or more prediction thresholds, and determining that the plurality of preliminary predictions meet a condition,
particularly
wherein the condition comprises the plurality of preliminary predictions being triggered consecutively, or at least a portion of the plurality of preliminary predictions being triggered.

10. The method of any of Claims 1 to 9, further comprising generating a prediction of a physiological condition of the user, the generating of the prediction comprising generating a first prediction relating to a first physiological condition and second prediction a second physiological condition, and reconciling the first and second predictions based on a rule.

11. The method of any of Claims 1 to 10, wherein the first prediction horizon is 60 minutes, and the second prediction horizon is under 60 minutes.

12. A system comprising:
one or more processors; and
one or more processor-readable media storing instructions which, when executed by the one or more processors, cause performance of:
obtaining one or more glucose concentration values measured from a user;
estimating, using the one or more glucose concentration values, a first blood glucose value of the user within a predetermined length of time;
obtaining a prediction horizon that is less than the predetermined length of time, a first threshold level, and a second threshold level;
estimating a second blood glucose value of the user within the prediction horizon based on the first blood glucose value and a first machine learning model trained to determine whether a blood glucose level of the user will be lower than the first threshold level within the prediction horizon, or based on the first blood glucose value and a second machine learning model trained to determine whether the blood glucose level of the user will be higher than the second threshold level within the prediction horizon; and
generating a prediction of a physiological condition of the user based at least on the estimated second blood glucose value.

13. The system of Claim 12, wherein:
the first machine learning model is configured to:
output a first raw indication of the second blood glucose value of the user based at least on at least one of the one or more glucose concentration values, the first blood glucose value of the user, the first threshold level, the prediction horizon, or a combination thereof; and
generate the prediction of the physiological condition of the user based on the first raw indication of the second blood glucose value of the user, wherein the physiological condition is hyperglycemia; and
the second machine learning model is configured to:
output a second raw indication of the second blood glucose value of the user based at least on the at least one of the one or more glucose concentration values, the first blood glucose value of the user, the second threshold level, the prediction horizon, or a combination thereof; and
generate the prediction of the physiological condition of the user based on the second raw indication of the second blood glucose value of the user, wherein the physiological condition is hypoglycemia.

14. The system of Claim 12 or 13, wherein:
the first machine learning model is further configured to generate the first raw indication based at least on an interpolation between the at least one of the one or more glucose concentration values, and the first blood glucose value of the user;
the second machine learning model is further configured to generate the second raw indication based at least on an interpolation between the at least one of the one or more glucose concentration values, and the first blood glucose value of the user;
the one or more processor-readable media storing instructions which, when executed by the one or more processors, further cause performance of providing, to the user, the prediction of the physiological condition of the user.

15. One or more non-transitory processor-readable media storing instructions which, when executed by one or more processors, cause performance of:
obtaining one or more glucose concentration values measured from a user;
applying, to the one or more glucose concentration values measured from the user, a first glucose prediction model for a first prediction horizon;
obtaining, based on applying the first glucose prediction model, a first predicted glucose value of the user; and
predicting a second predicted glucose value of the user for a second prediction horizon that is less than the first prediction horizon, based on the first predicted glucose value and at least one glucose concentration value of the one or more glucose concentration values.
